# EUROPEAN PATENT APPLICATION

(11) **EP 4 692 093 A1**
(43) Date of publication of application: **11.02.2026**
(21) Application number: 24781118.5
(22) Date of filing: 21.03.2024
(51) Int. Cl.: C07D 495/10, C07D 493/10, C07D 491/107, C07D 471/10, H10K 85/60, H10K 50/11

(54) **ORGANIC COMPOUND AND ORGANIC ELECTROLUMINESCENT DEVICE USING SAME**

(30) Priority: 24.03.2023 KR 20230038569
(71) Applicant: Solus Advanced Materials Co., Ltd., Iksan-si, Jeollabuk-do 54584 (KR)
(72) Inventor: CHO, Sukwon, Seongnam-si, Gyeonggi-do 13636 (KR); SIM, Jaeyi, Seongnam-si, Gyeonggi-do 13636 (KR); BAE, Hyungchan, Seongnam-si, Gyeonggi-do 13636 (KR); SHIN, Seongmin, Seongnam-si, Gyeonggi-do 13636 (KR)
(74) Representative: Office Freylinger
(86) International application number: PCT/KR2024/003555
(87) International publication number: WO 2024/205127

(57) **Abstract**

The present invention relates to: a novel compound having excellent carrier transport ability, light-emitting ability, and thermal stability; and an organic electroluminescent device comprising the novel compound in one or more organic layers and thus having improved properties of luminous efficiency, driving voltage, lifespan, etc.

## Description

### Technical Field

The present invention relates to a novel organic compound and an organic electroluminescent device using the same and, more specifically, to a novel compound with excellent electron transporting ability, and an organic electroluminescent device exhibiting improved characteristics, such as luminous efficiency, driving voltages, and lifespan, by containing the novel compound in one or more organic layers.

### Background Art

In an organic electroluminescent device, upon the application of voltage between two electrodes, holes from an anode and electrons from a cathode are injected into organic layers. The injected holes and electrons combine with each other to form excitons, and the excitons fall down to the ground state to emit light. Particularly, materials used for the organic layers may be classified into light emission materials, hole injection materials, hole transport materials, electron transport materials, electron injection materials, and the like according to the function thereof.

Materials for forming an emissive layer of the organic electroluminescent device may be classified into blue, green and red light emission materials according to the color of light emission. Additionally, yellow and orange light emission materials may be used as light emission materials for displaying better natural colors. Additionally, host/dopant-based light emission materials may be used as light emission materials to increase color purity and improve luminous efficiency through energy transfer.

Dopant materials may be classified into fluorescent dopants using organic materials and phosphorescent dopants using metal complex compounds containing heavy atoms, such as Ir and Pt. These phosphorescent materials can theoretically improve the luminous efficiency up to four times compared to fluorescent materials, so research has been conducted on phosphorescent host materials as well as phosphorescent dopants.

Until today, NPB, BCP, Alq₃, and the like have been widely known as materials for use in a hole injection layer, a hole transport layer, and an electron transport layer, and anthracene derivatives have been reported as the light emitting layer materials. In particular, among the light emitting layer materials, metal complex compounds containing Ir, such as Firpic, Ir(ppy)₃, (acac)Ir(btp)₂, and the like, which have advantages in terms of improving efficiency, are used as blue, green, and red phosphorescent dopant material, and 4,4-dicarbazollybiphenyl (CBP) is used as the phosphorescent host material.

However, conventional light emission materials have advantages in terms of light emission characteristics, but are not satisfactory in terms of lifespan of organic electroluminescent devices due to low glass transition temperatures and very poor thermal stability. Accordingly, there is a need to develop light emission materials with excellent performance.

### Disclosure of Invention

### Technical Problem

The present invention has been made to solve the above-mentioned problems, and an aspect of the present invention is to provide a novel compound, which can be used as a material for an organic layer of an organic electroluminescent device, specifically, a material for an electron transport layer, an auxiliary electron transport layer, or a material for an light emitting layer, an due to excellent thermal stability, carrier transport ability, and light emission capacity and the like.

Another aspect of the present invention is to provide an organic electroluminescent device exhibiting a low driving voltage, high luminous efficiency, and improved lifespan by containing the above-described novel compound.

Other purposes and advantages of the present disclosure will be clarified by following detailed description and claims.

### Solution to Problem

To achieve the above objectives, the present invention provides a compound represented by the following Chemical Formula 1: 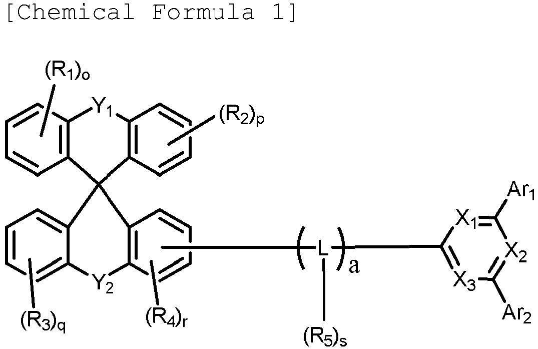
wherein in Chemical Formula 1,
X₁ to X₃ are the same as or different from each other and are each independently C(R₆) or N, provided that at least two of X₁ to X₃ are N,
Y₁ to Y₂ are the same as or different from each other and are each independently selected from the group consisting of O, S, NR₁₁,
Ar₁ and Ar₂ are the same as or different from each other and are each independently selected from the group consisting of a C₁-C₄₀ alkyl group, a C₂-C₄₀ alkenyl group, a C₂-C₄₀ alkynyl group, a C₃-C₄₀ cycloalkyl group, a heterocycloalkyl group having 3 to 40 ring atoms, a C₆-C₆₀ aryl group, a heteroaryl group having 5 to 60 ring atoms, a C₁-C₄₀ alkyloxy group, a C₆-C₆₀ aryloxy group, a C₃-C₄₀ alkylsilyl group, a C₆-C₆₀ arylsilyl group, a C₁-C₄₀ alkylboron group, a C₆-C₆₀ arylboron group, a C₆-C₆₀ arylphosphine group, a C₆-C₆₀ arylphosphine oxide group and a C₆-C₆₀ arylamine group, or combine with an adjacent group to form a fused ring;
R₁ to R₆ and R₁₁ are the same as or different from one another and are each independently selected from the group consisting of a hydrogen, a deuterium (D), a halogen, a cyano group, a nitro group, a C₁-C₄₀ alkyl group, a C₂-C₄₀ alkenyl group, a C₂-C₄₀ alkynyl group, a C₃-C₄₀ cycloalkyl group, a heterocycloalkyl group having 3 to 40 ring atoms, a C₆-C₆₀ aryl group, a heteroaryl group having 5 to 60 ring atoms, a C₁-C₄₀ alkyloxy group, a C₆-C₆₀ aryloxy group, a C₃-C₄₀ alkylsilyl group, a C₆-C₆₀ arylsilyl group, a C₁-C₄₀ alkylboron group, a C₆-C₆₀ arylboron group, a C₆-C₆₀ aryl phosphine group, a C₆-C₆₀ arylphosphine oxide group, and a C₆-C₆₀ arylamine group, or combine with an adjacent group to form a fused ring;
L is selected from the group consisting of a C₆ to C₁₈ arylene group and a heteroarylene group having 5 to 18 nuclear atoms,
a is an integer of 1 to 5, and where a is 2 or more, a plurality of L groups are the same as or different from each other,
o, p, q, and s are each independently an integer of 0 to 4,
r is an integer of 0 to 3, and
the arylene group, the heteroarylene group of L; and the alkyl group, alkenyl group, alkynyl group, aryl group, heteroaryl group, aryloxy group, alkyloxy group, cycloalkyl group, heterocycloalkyl group, alkylsilyl group, arylsilyl group, alkylboron group, arylboron group, arylphosphine group, arylphosphine oxide group, and arylamine group of Ar₁ to Ar₂, R₁ to R₆ and R₁₁ may be each independently substituted with at least one substituent selected from the group consisting of a deuterium (D), a halogen, a cyano group, a nitro group, a C₁ to C₄₀ alkyl group, a C₂ to C₄₀ alkenyl group, a C₂ to C₄₀ alkynyl group, a C₃ to C₄₀ cycloalkyl group, a heterocycloalkyl group having 3 to 40 ring atoms, a C₆ to C₆₀ aryl group, a heteroaryl group having 5 to 60 ring atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkylboron group, a C₆ to C₆₀ arylboron group, a C₆ to C₆₀ arylphosphine group, a C₆ to C₆₀ arylphosphine oxide group, and a C₆ to C₆₀ arylamine group, and wherein when the substituent is present in a plural number, they may be the same or different from each other.

In addition, the present invention also provides an organic electroluminescent device including: an anode, a cathode, and one or more organic layers disposed between the anode and the cathode, wherein at least one of the one or more organic layers includes the compound represented by Chemical Formula 1.

In such a case, the organic layer including the compound represented by Chemical Formula 1 may be selected from a light emitting layer, a light emitting auxiliary layer, a hole injection layer, a hole transport layer, an electron injection layer, a lifespan improvement layer, an electron transport layer, and an auxiliary electron transport layer. In such a case, the compound represented by Chemical Formula 1 may be included as a material for at least one of a phosphorescent host material of a light emitting layer, an electron transport layer and an auxiliary electron transport layer.

### EFFECTS OF THE INVENTION

According to an embodiment of the present invention, a compound represented by Chemical Formula 1 has excellent characteristics such as an electron transport ability, luminescence ability, heat resistance, and the like, and thereby is applicable as an organic layer material of an organic electroluminescent device.

In particular, when the compound represented by Chemical Formula 1 of the present invention is used as an electron transport layer material or an auxiliary electron transport layer material, it may exhibit high thermal stability, low driving voltage, rapid mobility, high current efficiency and long lifespan characteristics compared to conventional host materials or electron transport materials.

Accordingly, the organic electroluminescent device including the compound of Chemical Formula 1 may be significantly improved in aspects such as excellent light emitting performance, low driving voltage, long lifespan, and high efficiency, and thus may be effectively applied to full color display panels and the like.

Effects according to the present invention are not limited by the description exemplified above, and more diverse effects are included in the present specification.

### DETAILED DESCRIPTION OF THE INVENTION

Hereinafter, the present invention will be described in detail.

### <Novel organic compound>

The present invention provides a novel aryl-based compound having excellent thermal stability, carrier transport ability, and light emitting ability, for example, a polycyclic spiro-based compound.

The compound represented by Chemical Formula 1 includes a core having a spiro bonded structure with excellent conductivity, which contains at least two hetero atoms (e.g., O-O/S-S/O-S/S-NR'/NR'-NR"/O-NR'), and an electron withdrawing group (EWG) having excellent electron transport ability, and these are bonded through a linker (L) to form a basic skeleton structure.

Typically, in the light-emitting layer of an organic light-emitting device, electrons and holes combine to generate excitons. When the excitons generated as described above are not blocked from adjacent layers of the light emitting layer, the efficiency of the device is reduced. In addition, when holes generated in the hole injection layer and need to be present in the light emitting layer are moved to the electron injection layer, the efficiency and lifetime of the device are reduced. In this case, when the organic material constituting the auxiliary electron transport layer or the electron transport layer adjacent to the light emitting layer has high singlet energy, triplet energy, and low HOMO energy, the excitons and holes generated in the light emitting layer can be effectively blocked from moving to the electron transport layer, and particularly, such an effect can be more clearly seen in a device using a phosphorescent light emitting material than in a device using a fluorescent light emitting material.

In the present invention, to solve the above-described problems, a cyclic compound-based core having excellent conductivity is structurally distorted to have higher singlet energy and triplet energy, and at least one arylene/heteroarylene-based linker (L) is introduced between the above-described core and the EWG group having excellent electron transport ability, thereby increasing the stability of a molecule.

And in the present invention, by using a spiro-based skeleton as the core structure, it is possible to secure excellent thermal stability because it has a high glass transition temperature (Tg), and by introducing a triazine or pyrimidine, which is a functional group having strong electron withdrawing ability, to improve the electron transfer rate, it is possible to have a more suitable physicochemical property for electron injection and electron transport. In addition, the compound of Chemical Formula 1 has an electron donating effect by a non-covalent electron pair of a hetero atom (e.g., O or S) included in the polycyclic spiro-based core structure, and thus, when the compound is applied to an organic light emitting device, the light emitting efficiency of the device can be improved, and the durability and stability of the device can be improved, thereby effectively increasing the lifetime of the device. When the compound of Chemical Formula 1 is applied as a material of the electron transport layer or the auxiliary electron transport layer, electrons can be easily received from a cathode, thereby smoothly transferring the electrons to the light emitting layer, and thus, the driving voltage of the device can be lowered, and high efficiency and long lifetime can be induced.

Additionally, the compound represented by Chemical Formula 1 of the present invention has a higher triplet energy level than the light emitting layer (e.g., the emission layer), thus preventing excitons generated in the light emitting layer from diffusing (moving) to adjacent electron transport layers or hole transport layers. This results in an increased number of excitons contributing to luminescence within the light emitting layer, improving device luminescence efficiency, durability, and stability, thereby extending device lifespan. Most of the developed materials exhibit physical properties that enable low-voltage operation, further improving device longevity.

As described above, when the compound represented by Chemical Formula 1 of the present invention may be applied as an organic layer material of an organic electroluminescent device, preferably a light-emitting layer material (a blue, a green and/or a red phosphorescent host material), an electron transport/injection layer material, a hole transport/injection layer material, a light-emitting auxiliary layer material, an auxiliary electron transport layer, and/or a lifespan enhancement layer material, the performance and lifetime characteristics of the organic electroluminescent device can be significantly improved. In particular, when the compound of the present invention is used as a material for an electron transport layer or an electron transport auxiliary layer, excellent improvements in device efficiency, driving voltage, and lifespan characteristics can be expected. As a result, such an organic electroluminescent device including the compound of the above chemical formula can maximize the performance of a full color organic light emitting panel.

According to the present invention, the compound represented by Chemical Formula 1 has a polycyclic spirobixanthene-based core with excellent conductivity, and a nitrogen-containing heteroaromatic ring (e.g., an azine, a X₁ to X₃-containing ring) having excellent electron withdrawing group-(EWG) properties with excellent electron transport capability, and a linker (L) is bonded between them to form a basic skeleton structure.

The polycyclic spiro-core structure includes at least two heteroatoms (e.g., a Y₁ and Y₂-containing ring). As one example of the polycyclic spiro core structure, Y₁ and Y₂ may be the same as or different from each other, and may each independently be selected from the group consisting of O, S and NR₁₁.

Herein, R₁₁ may each independently be selected from the group consisting of a hydrogen, a deuterium (D), a halogen, a cyano group, a nitro group, a C₁ to C₄₀ alkyl group, a C₂ to C₄₀ alkenyl group, a C₂ to C₄₀ alkynyl group, a C₃ to C₄₀ cycloalkyl group, a heterocycloalkyl group having 3 to 40 nuclear atoms, a C₆ to C₆₀ aryl group, a heteroaryl group having 5 to 60 nuclear atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkylboron group, a C₆ to C₆₀ arylboron group, a C₆ to C₆₀ arylphosphine group, a C₆ to C₆₀ arylphosphine oxide group, and a C₆ to C₆₀ arylamine group, or combine with an adjacent group to form a fused ring. In this case, when R₁₁ may be plural in number, the plurality of R₁₁ may be the same as or different from each other. Specifically, R₁₁ may preferably be selected from: a hydrogen, a deuterium (D), a C₁ to C₄₀ alkyl group, a C₆ to C₆₀ aryl group, and a heteroaryl group having 5 to 60 nuclear atoms.

In an embodiment of the present invention, the polycyclic spiro-core structure (e.g., Y₁ ~ Y₂-containing ring) may be more specifically embodied as any one of the following structural formulae. However, it is not limited thereto. 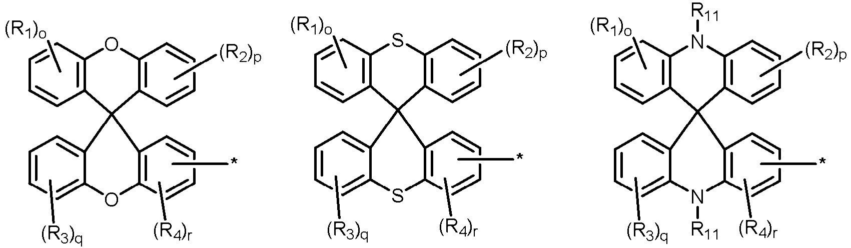 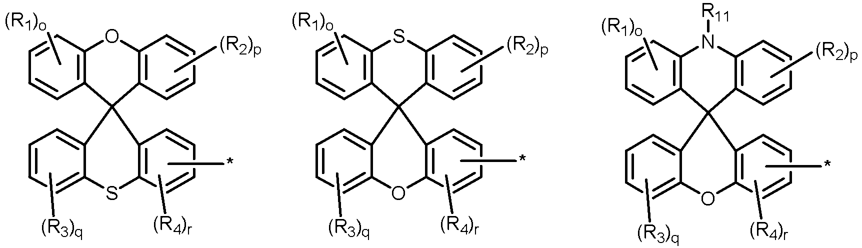 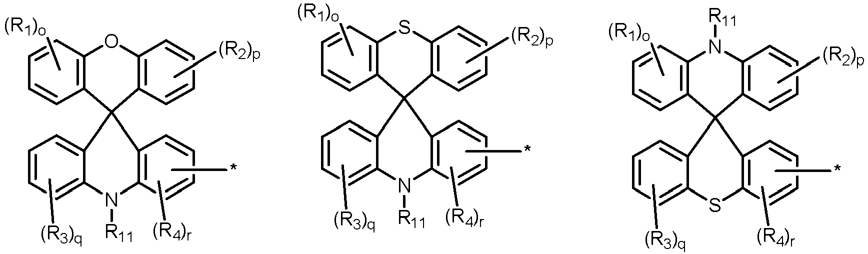
wherein the formulas,
* indicates a site where a bond with Chemical Formula 1 is made,
R₁₁ may be selected from the group consisting of a hydrogen, a deuterium (D), a C₁-C₄₀ alkyl group, a C₆-C₆₀ aryl group, and a heteroaryl group having 5 to 60 ring atoms, and
R₁ to R₄, o, p, q and r are each as defined in Chemical Formula 1.

The polycyclic spiro-core structure (e.g., Y₁ ~ Y₂-containing ring) according to the present invention may each be substituted with R₁ to R₄ as various substituents. Such R₁ to R4 may be the same as or different from each other, and may each independently be selected from the group consisting of a hydrogen, a deuterium (D), a halogen, a cyano group, a nitro group, a C₁ to C₄₀ alkyl group, a C₂ to C₄₀ alkenyl group, a C₂ to C₄₀ alkynyl group, a C₃ to C₄₀ cycloalkyl group, a heterocycloalkyl group having 3 to 40 nuclear atoms, a C₆ to C₆₀ aryl group, a heteroaryl group having 5 to 60 nuclear atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkylboron group, a C₆ to C₆₀ arylboron group, a C₆ to C₆₀ arylphosphine group, a C₆ to C₆₀ arylphosphine oxide group, and a C₆ to C₆₀ arylamine group, or combine with an adjacent group to form a fused ring. In this case, when R₁ to R₄ may be plural in number, the plurality of R₁ to R₄ may be the same as or different from each other. Specifically, R₁ to R₄ may be the same as or different from each other, and may preferably be selected from: a hydrogen, a deuterium (D), a cyano group, a C₁ to C₄₀ alkyl group, a C₆ to C₆₀ aryl group, and a heteroaryl group having 5 to 60 nuclear atoms, or combine with adjacent group to form a fused ring.

Herein, o, p, and q may each independently be an integer of 0 to 4, and r may be an integer of 0 to 3. When o is 0, R₁ may be a hydrogen, and when o is an integer of 1 to 3, R₁ may have the aforementioned substituents other than hydrogen. p, q, and r may also be equally applied.

In Chemical Formula 1 according to the present invention, a nitrogen-containing heteroaromatic ring (e.g., an azine, X₁ to X₃-containing ring) having an EWG property with excellent electron transport ability is connected via a linker (L) to one side phenyl ring of the polycyclic spiro-core structure (e.g., Y1~Y2 containing ring) .

The nitrogen-containing heterocycle (e.g., X₁ to X₃-containing ring) may be a monocyclic or polycyclic heteroaryl group (e.g., azine) containing at least two nitrogen atoms. For an example of the nitrogen-containing heteroaromatic ring (e.g., X₁ to X₃-containing ring), X₁ to X₃ may be the same as or different from each other, and may each independently be C(R₆) or N, provided that at least two of X₁ to X₃ are N. For a specific example, X₁ to X₃ includes two to three nitrogen atoms (N). As such, since the heterocycle containing two to three nitrogen atoms (N) is included, more excellent electron absorption characteristics may be exhibited, which is advantageous for electron injection and transport.

Herein, R₆ may be selected from the group consisting of a hydrogen, a deuterium (D), a C₁ to C₄₀ alkyl group, a C₂ to C₄₀ alkenyl group, a C₂ to C₄₀ alkynyl group, a C₃ to C₄₀ cycloalkyl group, a heterocycloalkyl group having 3 to 40 nuclear atoms, a C₆ to C₆₀ aryl group, a heteroaryl group having 5 to 60 nuclear atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkylboron group, a C₆ to C₆₀ arylboron group, a C₆ to C₆₀ arylphosphine group, a C₆ to C₆₀ monoarylphosphinyl group, a C₆ to C₆₀ diarylphosphinyl group, a C₆ to C₆₀ arylamine group, a C₅ to C₆₀ arylheteroarylamine group, and a heteroarylamine group having 5 to 60 nuclear atoms, or combine with an adjacent group to form a fused ring. In this case, when R₅ may be plural in number, the plurality of R₆ may be the same as or different from each other. Specifically, R₆ may preferably be selected from: a hydrogen, a deuterium (D), a C₁ to C₄₀ alkyl group, a C₆ to C₆₀ aryl group, and a heteroaryl group having 5 to 60 nuclear atoms.

In an embodiment of the present invention, the nitrogen-containing heterocycle (e.g., X₁ to X₃-containing ring) may be more specifically embodied as any one of the following structural formulas. However, it is not limited thereto. 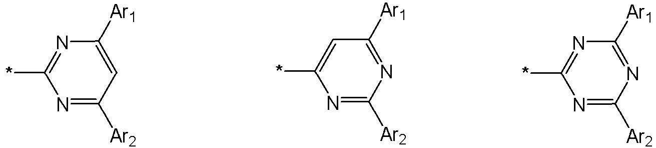 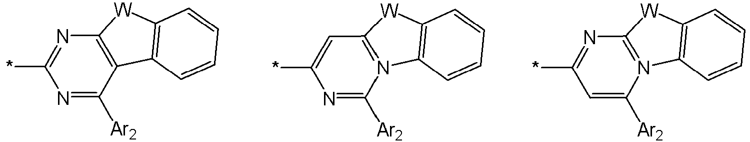 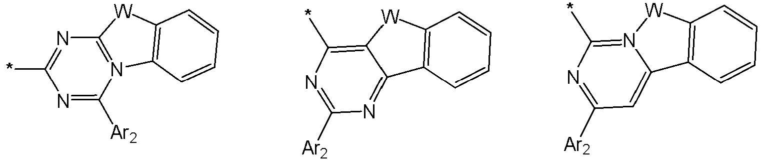 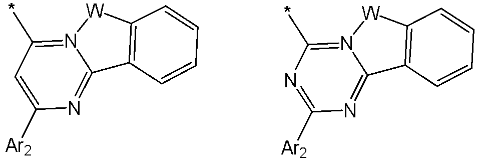
wherein the formulas,
* indicates a site where a bond with Chemical Formula 1 is made,
W is O or S, and
Ar₁ and Ar₂ are each as defined in Chemical Formula 1.

The nitrogen-containing heterocycle (e.g., X₁ to X₃-containing ring) according to the present invention may each be substituted with Ar₁ to Ar₂ as various substituents. Such Ar₁ and Ar₂ may be the same as or different from each other and may each independently be selected from: a C₁ to C₄₀ alkyl group, a C₂ to C₄₀ alkenyl group, a C₂ to C₄₀ alkynyl group, a C₃ to C₄₀ cycloalkyl group, a heterocycloalkyl group having 3 to 40 nuclear atoms, a C₆ to C₆₀ aryl group, a heteroaryl group having 5 to 60 nuclear atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkylboron group, a C₆ to C₆₀ arylboron group, a C₆ to C₆₀ arylphosphine group, a C₆ to C₆₀ arylphosphine oxide group, and a C₆ to C₆₀ arylamine group, or combine with an adjacent group to form a fused ring. specifically, Ar₁ and Ar₂ may be each independently selected from: a C₆ to C₆₀ aryl group, and a heteroaryl group having 5 to 60 nuclear atoms, and preferably a C₆ to C₄₀ aryl group, or a heteroaryl group having 5 to 40 nuclear atoms.

In an embodiment of the present invention, Ar₁ and Ar₂ may be the same as or different from each other and may each independently be more specifically embodied as any one of the following structural formulas. However, it is not limited thereto. 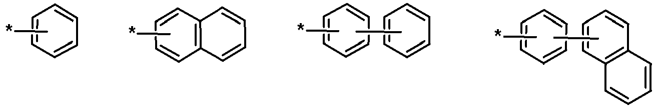 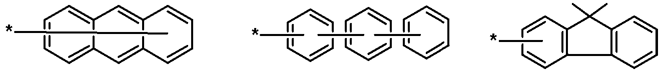 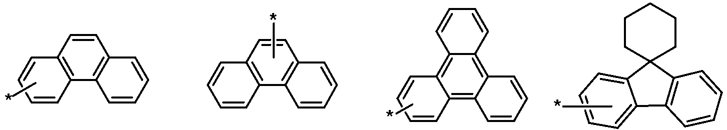 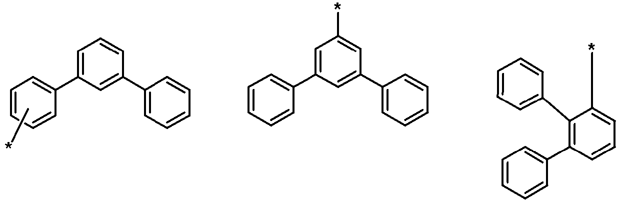 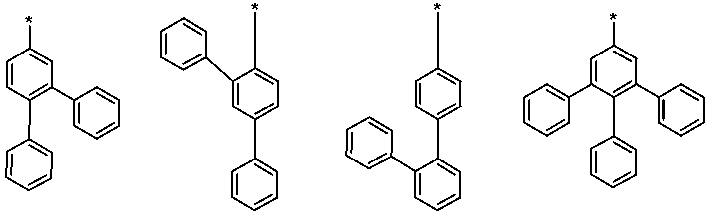 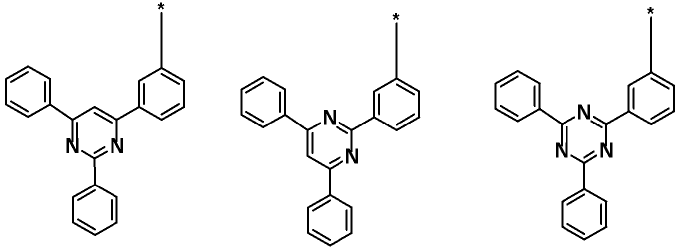 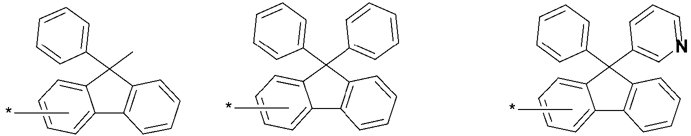 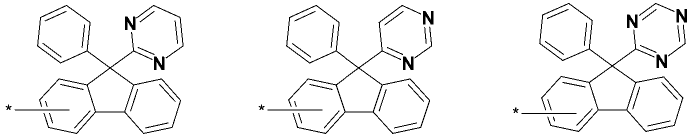 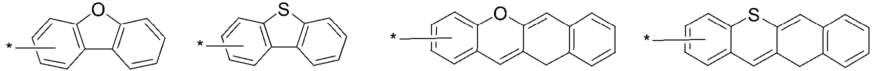 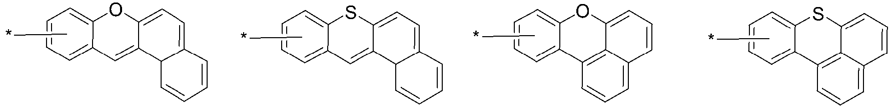 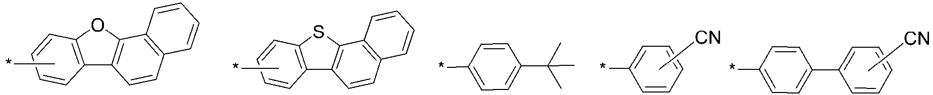
wherein the formulas,
* indicates a site where a bond with Chemical Formula 1 is made. In addition, although not shown in the above structural formulas, at least one substituent known in the art (e.g., the same as the definition of R₅) may be substituted.

In the compound represented by Chemical Formula 1 according to the present invention, a polycyclic spiro core (e.g., a ring containing Y₁-Y₂) with excellent conductivity and a nitrogen-containing heteroaromatic ring (e.g., azine, a ring containing X₁-X₃) with excellent electron transport ability and EWG characteristics may be connected through a separate linker (e.g., L). As such, when a separate linker (L) is present between the polycyclic spiro core and the nitrogen-containing heteroaromatic ring, a HOMO region may be expanded to give a benefit to a HOMO-LUMO distribution, and charge transfer efficiency may be increased through an appropriate overlap of HOMO-LUMO.

The linker (e.g., L) are not particularly limited, and may be a common divalent group linker known in the art. Specifically, L may be a single bond or may be selected from the group consisting of a C₆ to C₁₈ arylene group and a heteroarylene group having 5 to 18 nuclear atoms. More specifically, L may be selected from the group consisting of a C₆ to C₁₂ arylene group and a heteroarylene group having 5 to 12 nuclear atoms. Here, the number of linkers (a) is an integer of 1 to 5, and specifically, it is preferable to be 1 to 3. When a is 1 to 5, a plurality of Ls may be the same as or different from each other.

Specific examples of the arylene group may include, for example, a phenylene group, a biphenylene group, a naphthylene group, an anthracenylene group, an indenylene group, a pyrantrenylene group, a carbazolylene group, a thiophenylene group, an indolylene group, a purinylene group, a quinolinylene group, a pyrrolylene group, an imidazolylene group, an oxazolylene group, a thiazolylene group, a pyridinylene group, a pyrimidinylene group and the like. More specifically, L may be preferably selected from: a phenylene group, and a biphenylene group. Also, specific examples of the heteroarylene group may include, for example, a pyrrole-based moiety, a furan-based moiety, a thiophene-based moiety, a pyridine-based moiety, a pyrimidine-based moiety, a pyrazine-based moiety, a triazine-based moiety, a dibenzofuran-based moiety, a dibenzothiophene-based moiety, and/or a dibenzoselenophenone-based moiety.

In an embodiment of the present invention, L may be a linker selected from the following structural formulas. 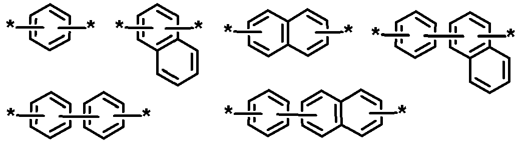 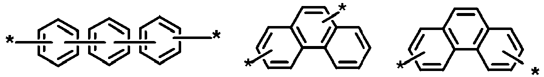 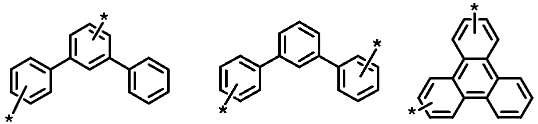 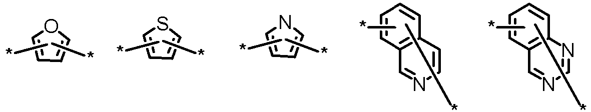 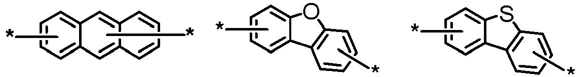
wherein the formulas,
* indicates a site where a bond with Chemical Formula 1 is made. In addition, although not shown in the above structural formulas, at least one substituent known in the art (e.g., the same as the definition of R₅) may be substituted.

The linker (e.g., L) according to the present invention may each be substituted with R₅ as various substituents. Such R₅ may be selected from the group consisting of a hydrogen, a deuterium (D), a C₁ to C₄₀ alkyl group, a C₂ to C₄₀ alkenyl group, a C₂ to C₄₀ alkynyl group, a C₃ to C₄₀ cycloalkyl group, a heterocycloalkyl group having 3 to 40 nuclear atoms, a C₆ to C₆₀ aryl group, a heteroaryl group having 5 to 60 nuclear atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkylboron group, a C₆ to C₆₀ arylboron group, a C₆ to C₆₀ arylphosphine group, a C₆ to C₆₀ monoarylphosphinyl group, a C₆ to C₆₀ diarylphosphinyl group, a C₆ to C₆₀ arylamine group, a C₅ to C₆₀ arylheteroarylamine group, and a heteroarylamine group having 5 to 60 nuclear atoms, or combine with an adjacent group to form a fused ring. In this case, when R₅ may be plural in number, the plurality of R₅ may be the same as or different from each other. Specifically, R₅ may preferably be selected from: a hydrogen, a deuterium (D), a C₁ to C₄₀ alkyl group, a C₆ to C₆₀ aryl group, and a heteroaryl group having 5 to 60 nuclear atoms.

Herein, s may be an integer of 0 to 4. When s is 0, R₅ may be a hydrogen, and when s is an integer of 1 to 4, R₅ may have the aforementioned substituents other than hydrogen.

In the above Chemical Formula 1, the arylene group, the heteroarylene group of L; and the alkyl group, alkenyl group, alkynyl group, aryl group, heteroaryl group, aryloxy group, alkyloxy group, cycloalkyl group, heterocycloalkyl group, alkylsilyl group, arylsilyl group, alkylboron group, arylboron group, arylphosphine group, arylphosphine oxide group, and arylamine group of Ar₁ to Ar₂, R₁ to R₆ and R₁₁ may be optionally each independently substituted with at least one substituent selected from the group consisting of: a deuterium (D), a halogen, a cyano group, a nitro group, a C₁ to C₄₀ alkyl group, a C₂ to C₄₀ alkenyl group, a C₂ to C₄₀ alkynyl group, a C₃ to C₄₀ cycloalkyl group, a heterocycloalkyl group having 3 to 40 ring atoms, a C₆ to C₆₀ aryl group, a heteroaryl group having 5 to 60 ring atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkylboron group, a C₆ to C₆₀ arylboron group, a C₆ to C₆₀ arylphosphine group, a C₆ to C₆₀ arylphosphine oxide group, and a C₆ to C₆₀ arylamine group, and provided that when the substituent is present in a plural number, they are optionally the same or different from each other.

In an embodiment of the present invention, the compound represented by Chemical Formula 1 may be more specifically represented by any one of the following Chemical Formulas 2 to 10 according to the type of the heteroatoms introduced into the polycyclic spiro core structure. However, it is not limited thereto. 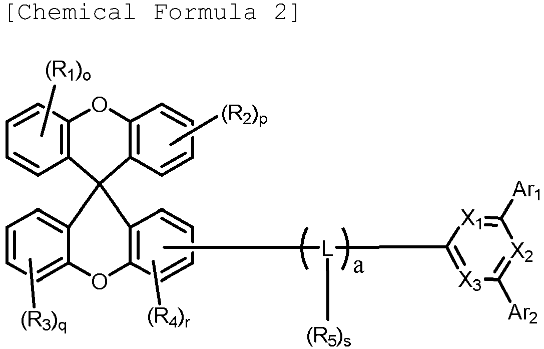 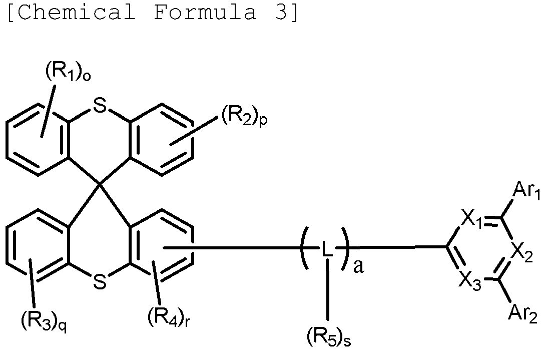 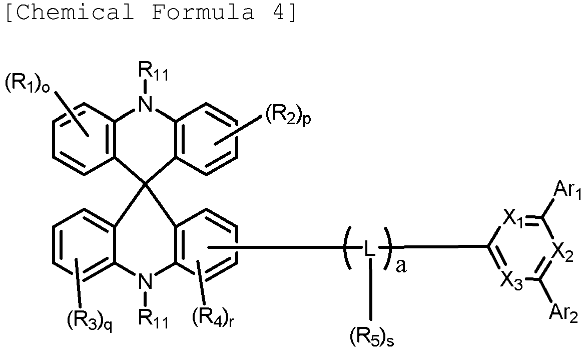 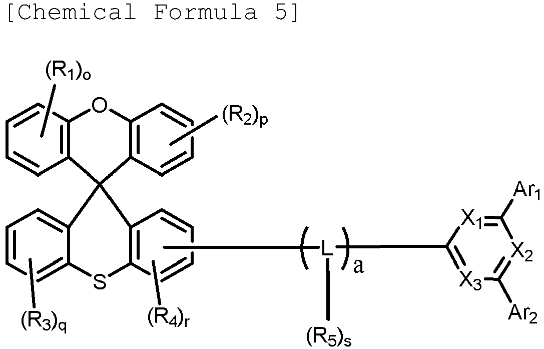 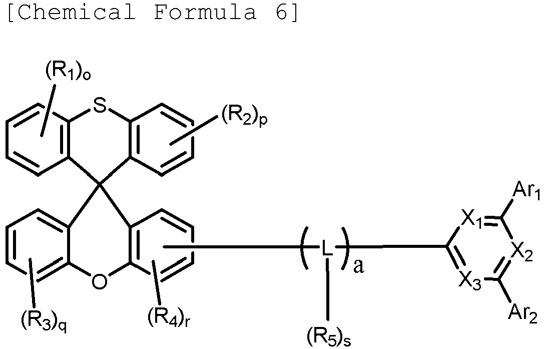 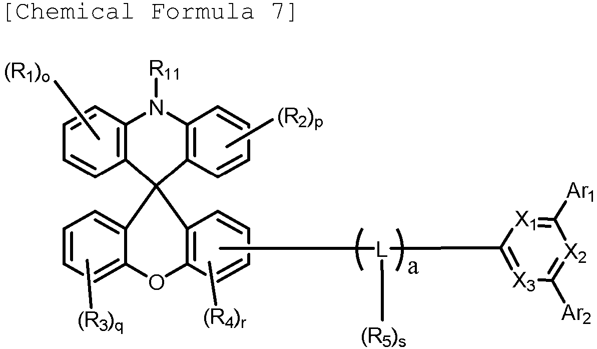 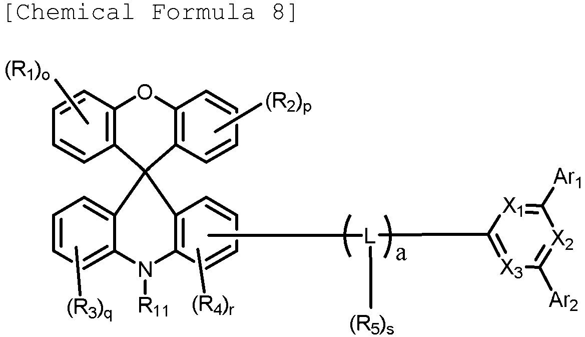 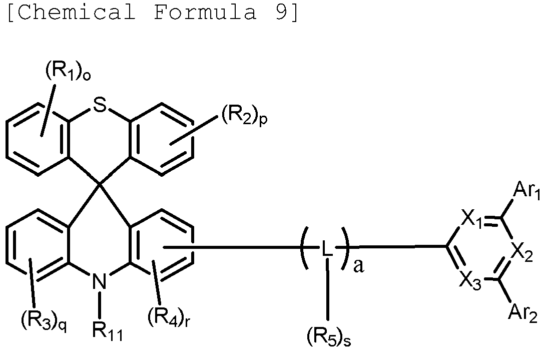 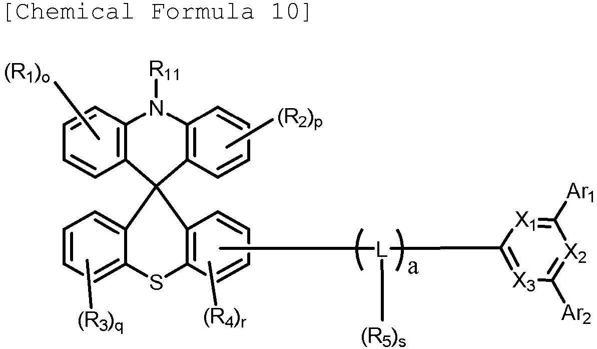
wherein the formulas,
X₁ to X₃, Ar₁ to Ar₂, L, R₁ to R₅, R₁₁, o, p, q, r, s and a are the same as defined in Chemical Formula 1.

In another embodiment of the present invention, the compound represented by Chemical Formula 1 may be more specifically represented by any one of the following Chemical Formulas 11 to 29 according to the type of the nitrogen-containing heterocycle (e.g., X₁ to X₃-containing ring). However, it is not limited thereto. 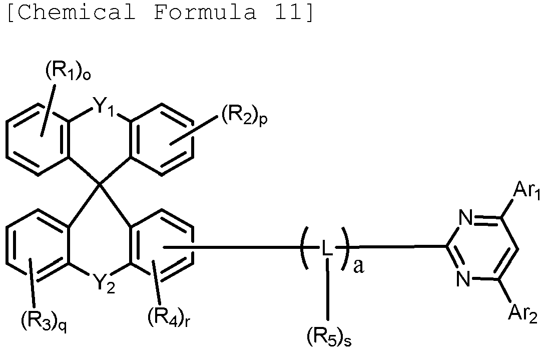 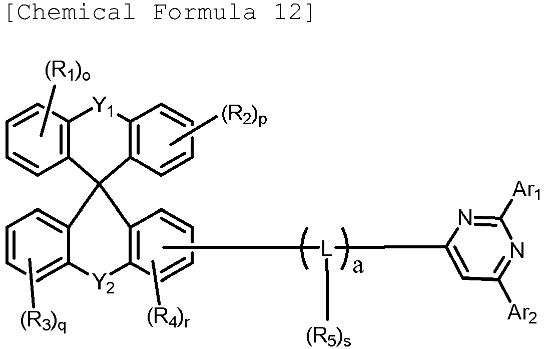 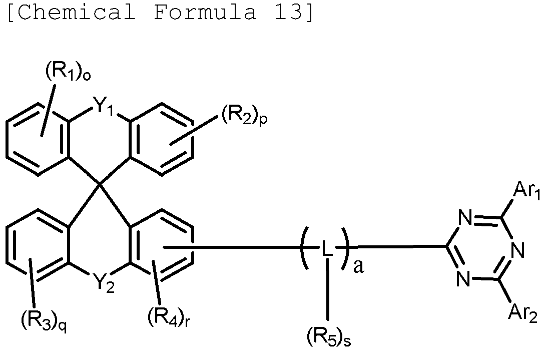 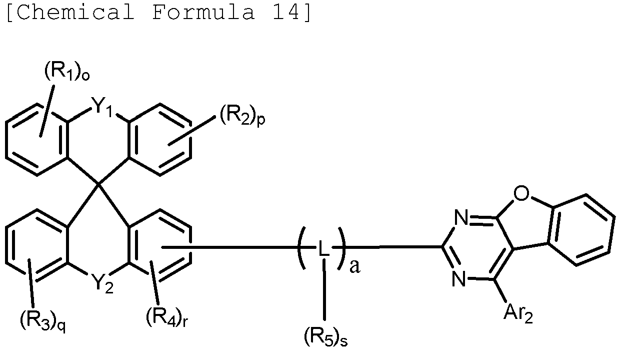 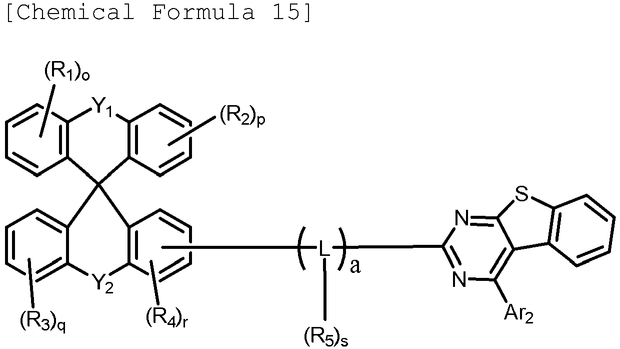 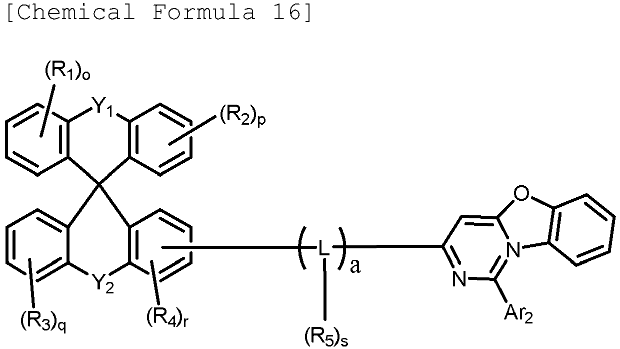 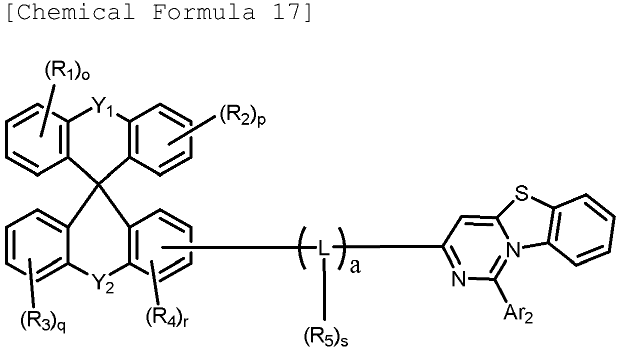 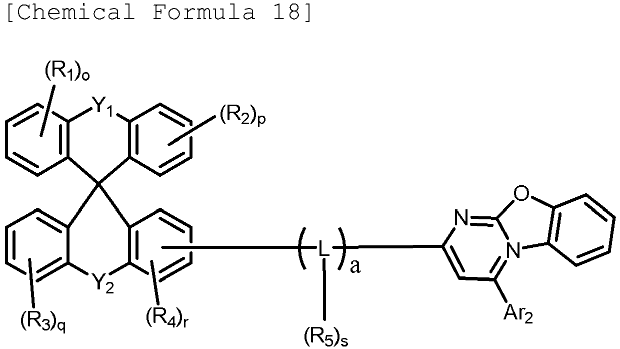 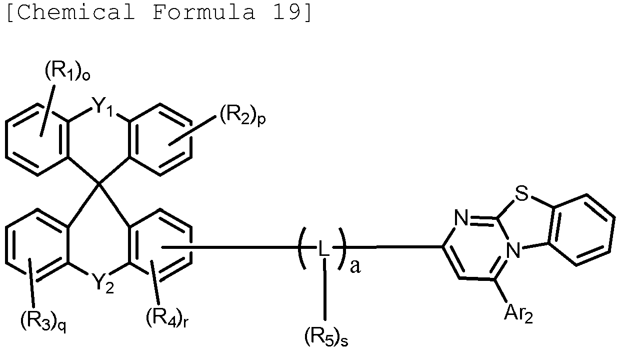 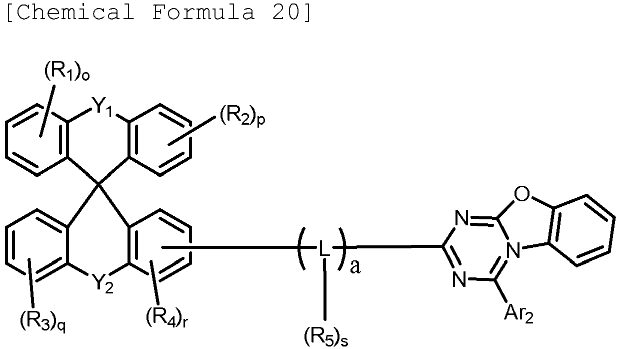 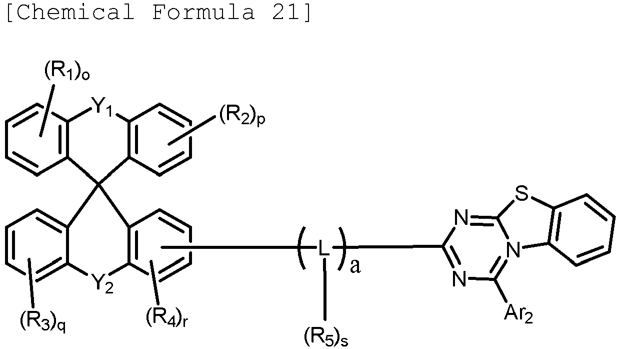 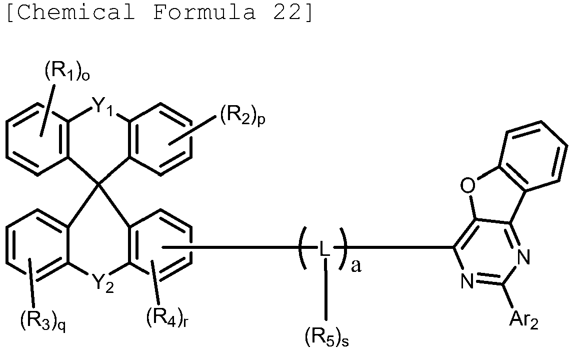 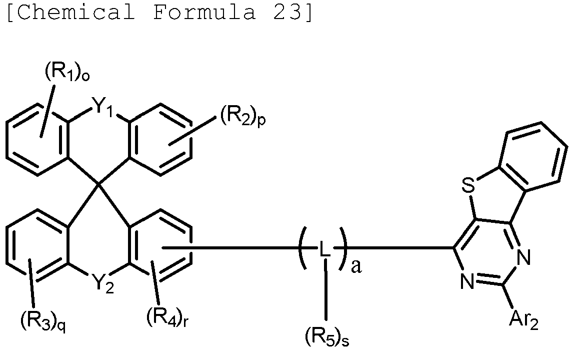 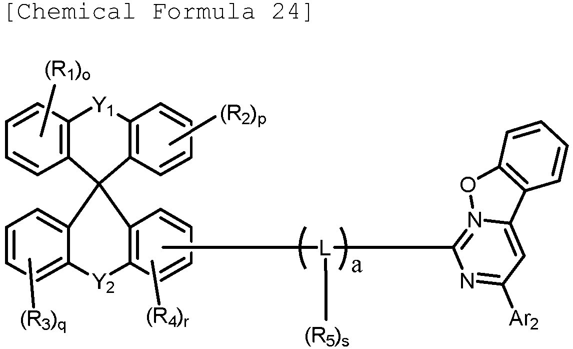 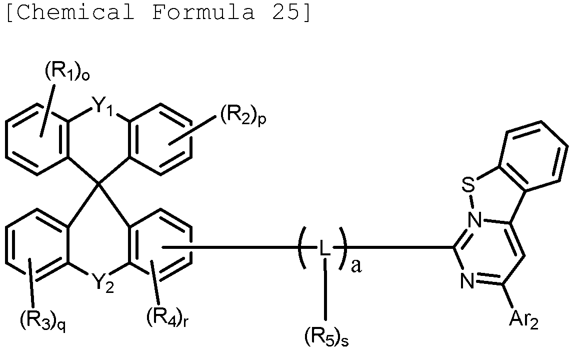 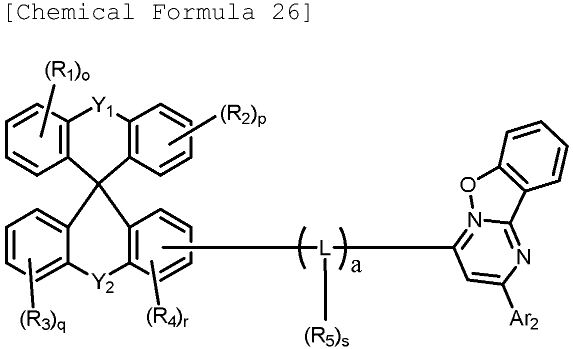 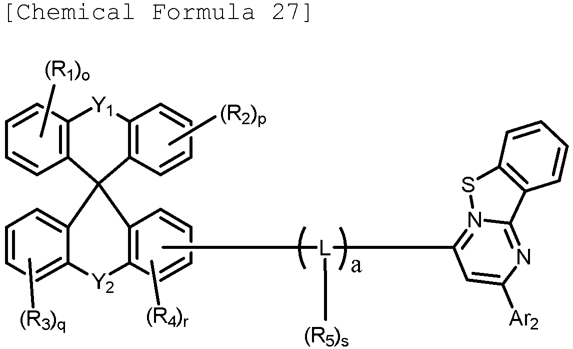 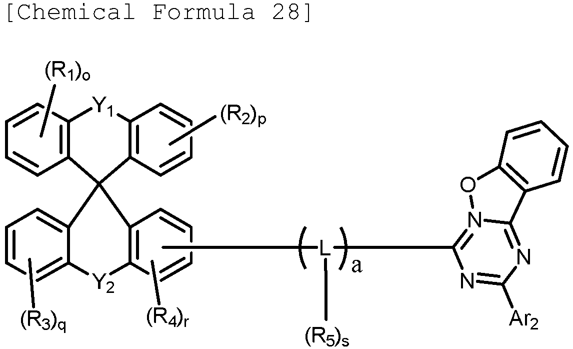 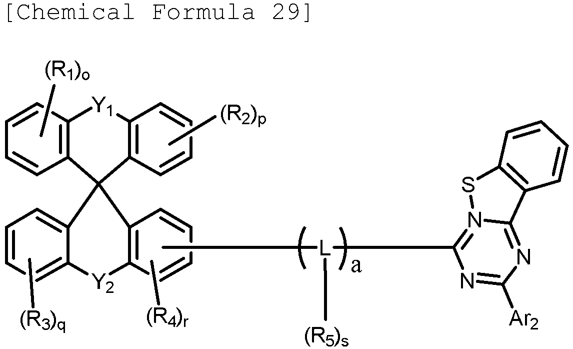
wherein the formulas,
Y₁ to Y₂, Ar₁ to Ar₂, L, R₁ to R₅, o, p, q, r, s and a are the same as defined in Chemical Formula 1.

In another embodiment of the present invention, the compound represented by Chemical Formula 1 may be more specifically represented by any one of the following Chemical Formulas 30 to 37 according to the bonding position of the fused ring formed on the polycyclic spiro core. However, it is not limited thereto. 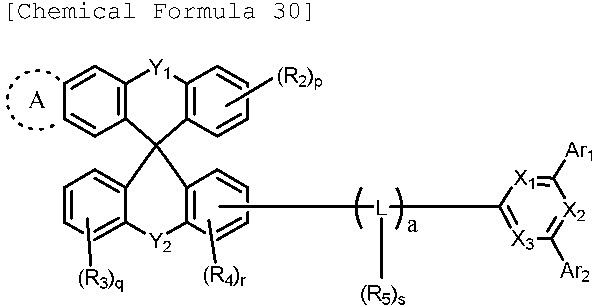 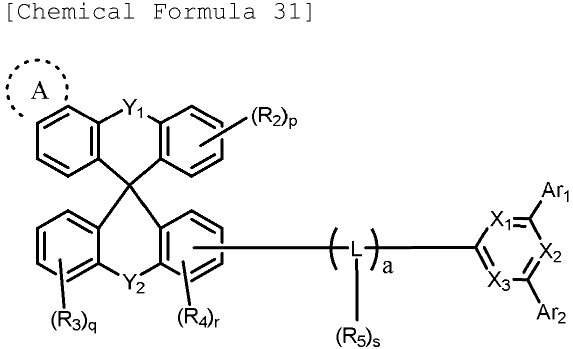 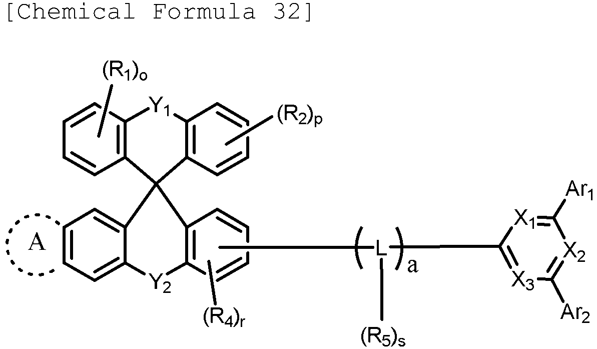 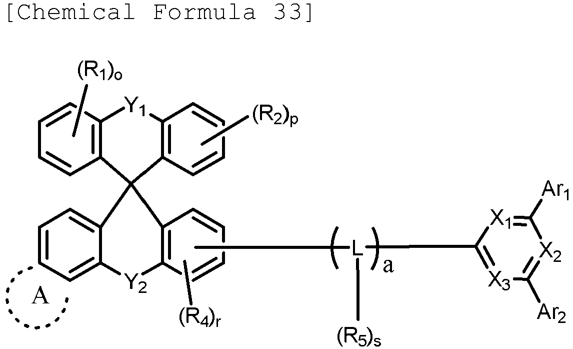 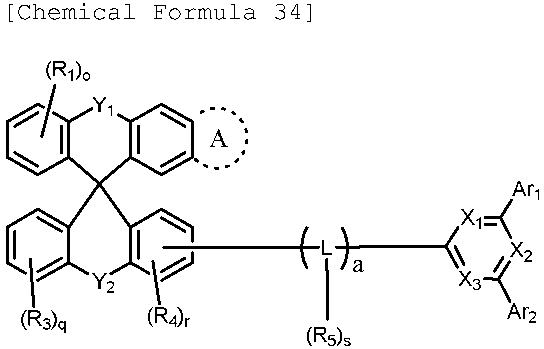 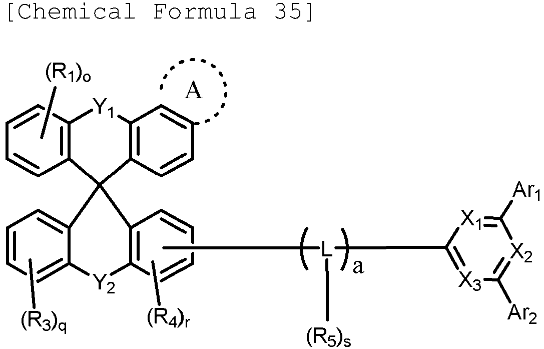 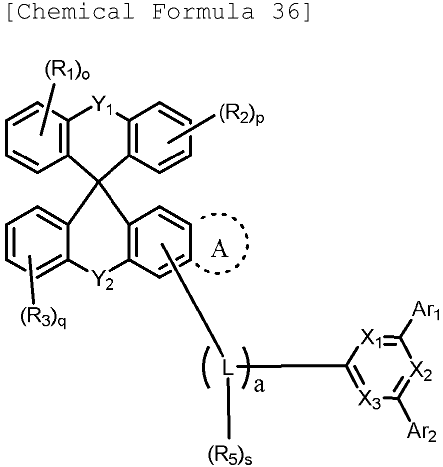 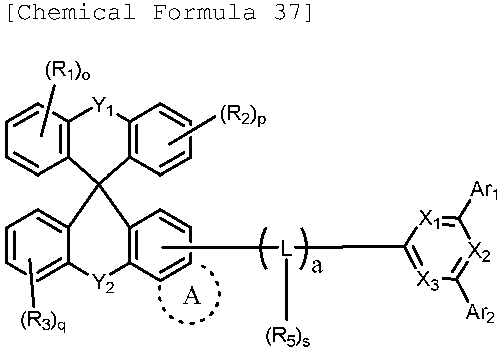
wherein the formulas,
Ring A may be a conventional hydrocarbon-based ring or a hydrocarbon-based ring containing at least one heteroatom known in the art, and may be in a form in which they are condensed, fused, bridged, or spirocyclic bonded to other rings (e.g., core structures) adjacent to each other. For example, the Ring A may be selected from the group consisting of: a monocyclic or polycyclic alicyclic ring, a monocyclic or polycyclic heteroalicyclic ring, a monocyclic or polycyclic aromatic ring, or a monocyclic or polycyclic heteroaromatic ring. Specifically, the Ring A may be preferably an aromatic ring of C₆ to C₁₈, or a heteroaromatic ring having 5 to 18 nuclear atoms.
X₁ to X₃, Y₁ to Y₂, Ar₁ to Ar₂, L, R₁ to R₅, o, p, q, r, s and a are the same as defined in Chemical Formula 1.

In another embodiment of the present invention, the compound represented by Chemical Formula 1 may be more specifically represented by any one of the following Chemical Formulas 38 to 40 according to the bonding position of the nitrogen-containing heterocycle. However, it is not limited thereto. 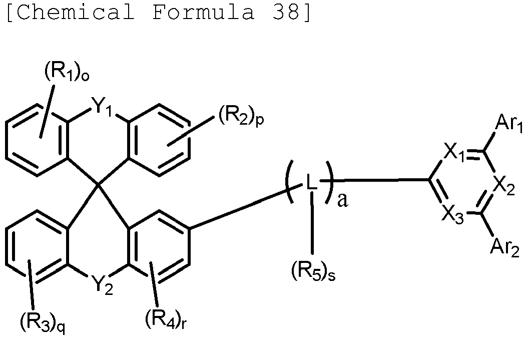 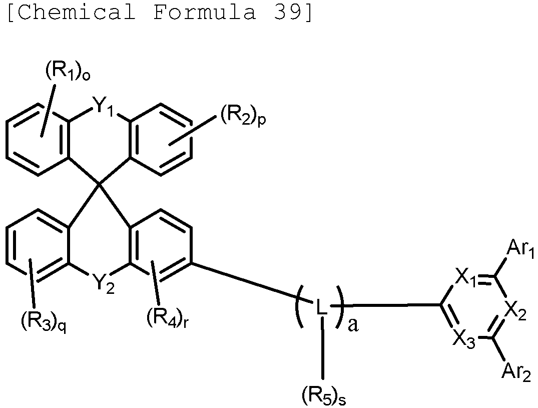 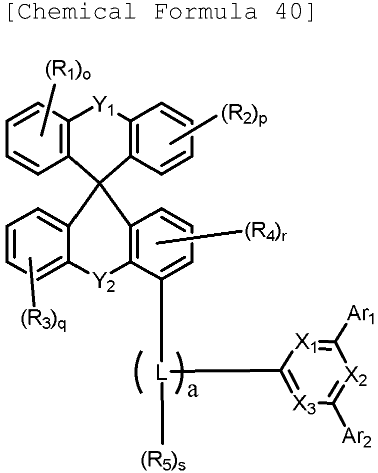
wherein the formulas,
X₁ to X₃, Y₁ to Y₂, Ar₁ to Ar₂, L, R₁ to R₅, o, p, q, r, s and a are the same as defined in Chemical Formula 1.

In another embodiment of the present invention, the compound represented by Chemical Formula 1 may be more specifically represented by any one of the following Chemical Formulas 41 to 42 according to type of the linker (L). However, it is not limited thereto. 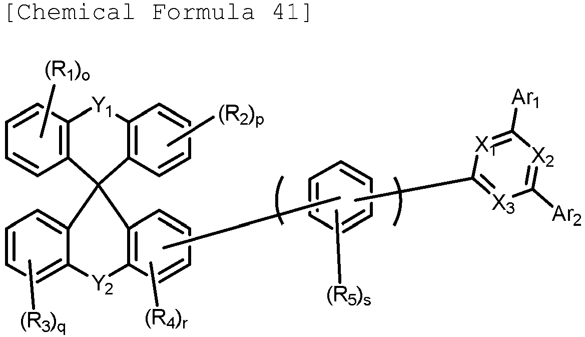 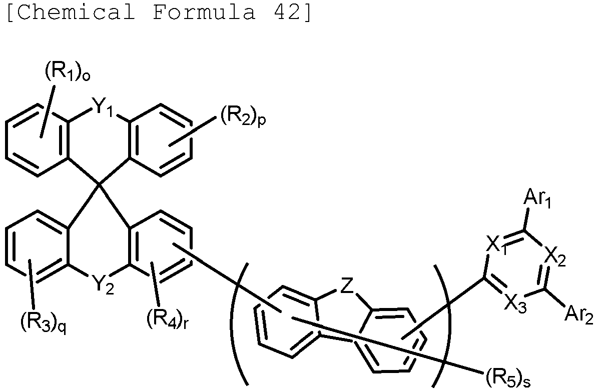
wherein the formulas,
Z is O or S, and
X₁ to X₃, Y₁ to Y₂, Ar₁ to Ar₂, R₁ to R₅, o, p, q, r, s and a are the same as defined in Chemical Formula 1.

The compound represented by Chemical Formula 1 of the present invention described above may be further embodied as compounds represented by the following compounds represented by, for example, 1 to 100. However, the compound represented by Chemical Formula 1 of the present invention is not limited to those exemplified below.

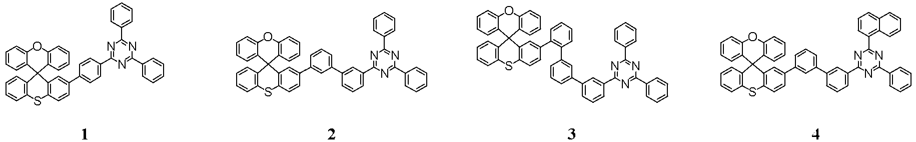

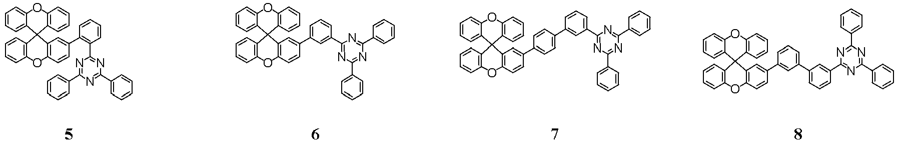

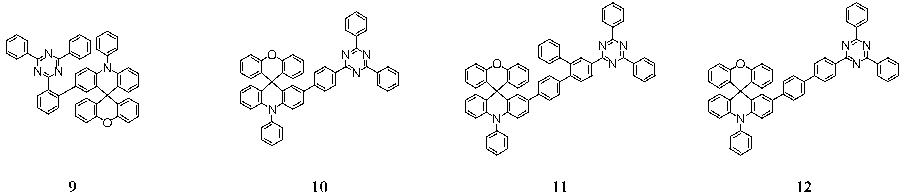

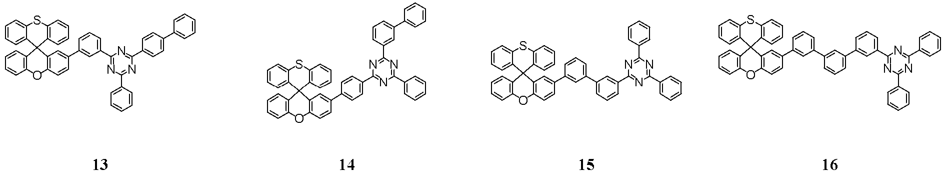

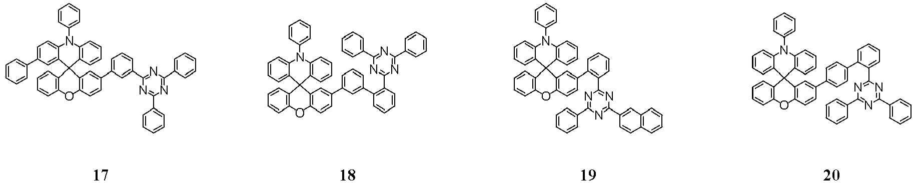

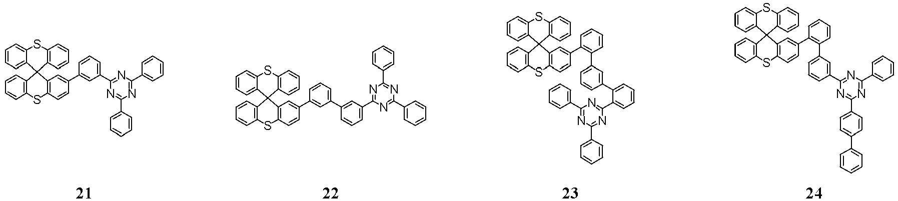

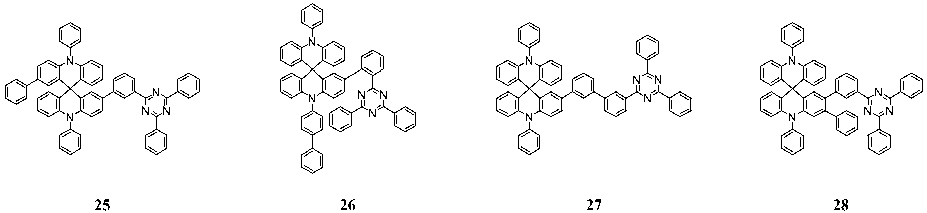

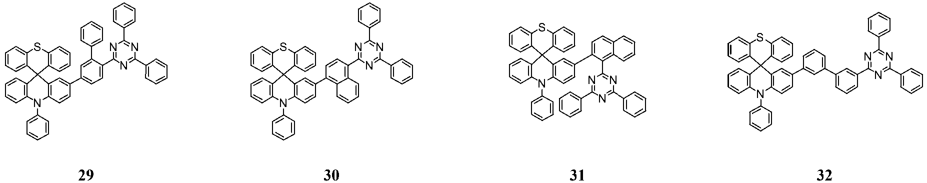

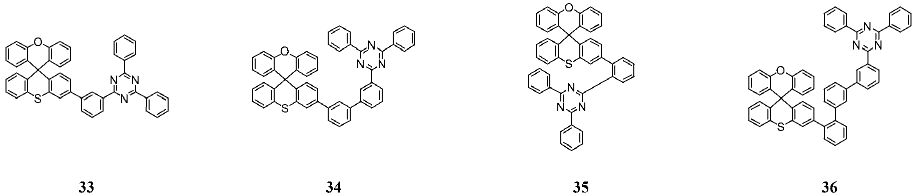

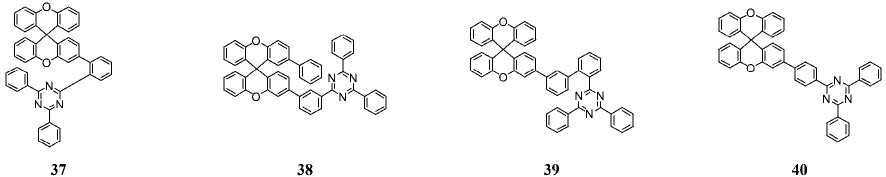

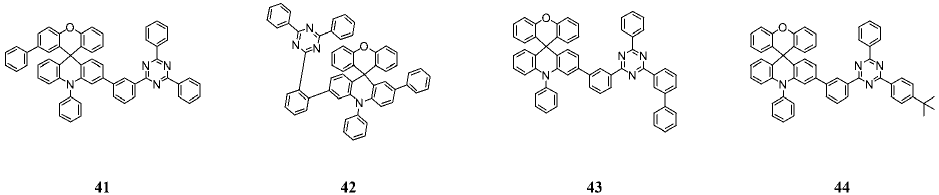

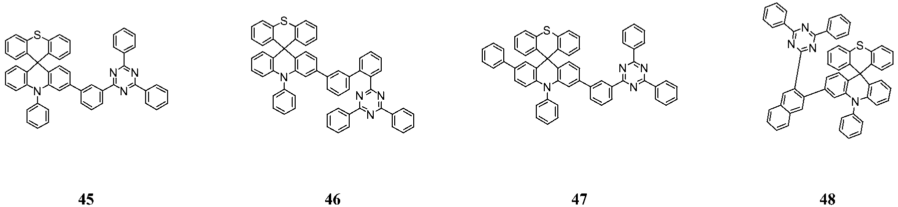

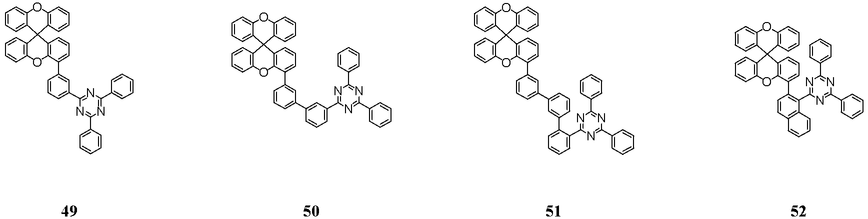

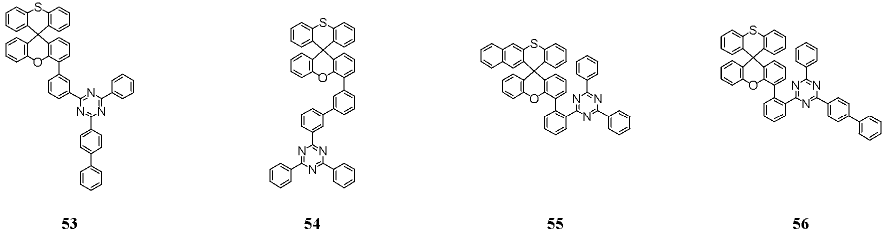

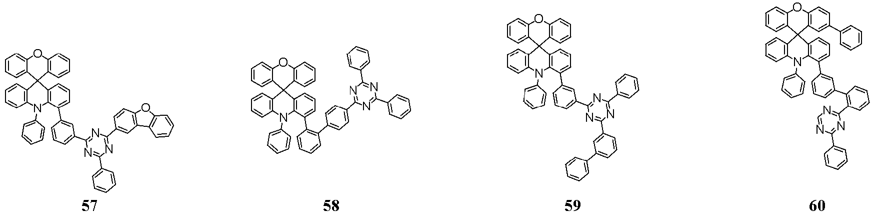

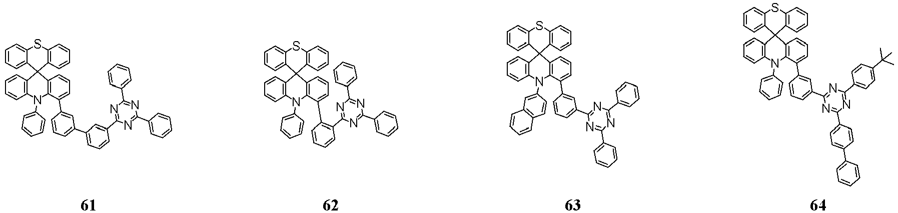

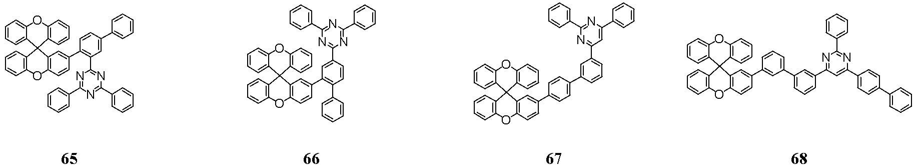

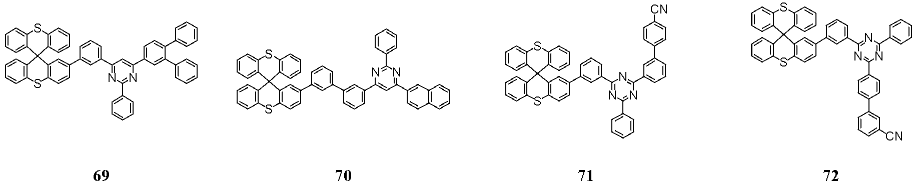

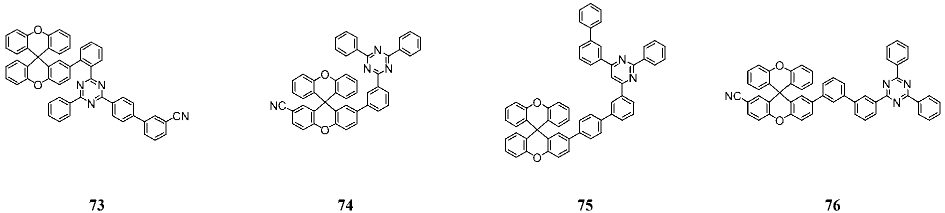

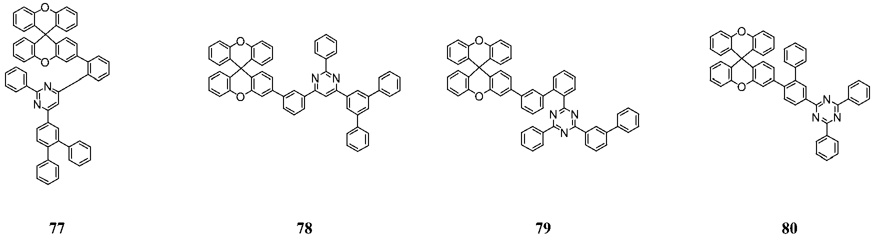

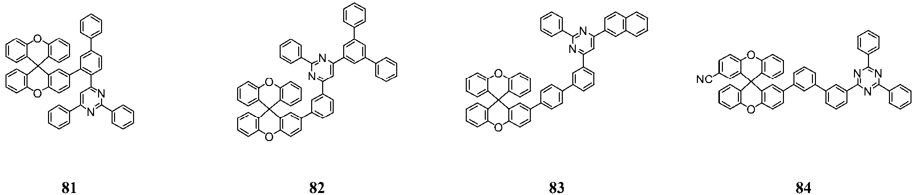

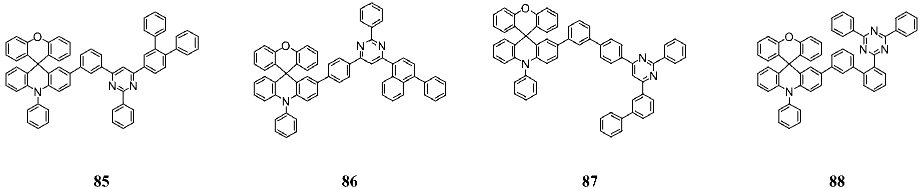

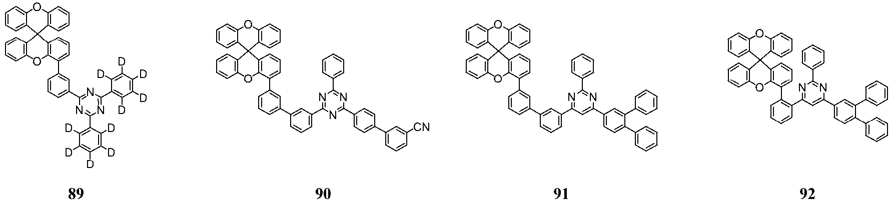

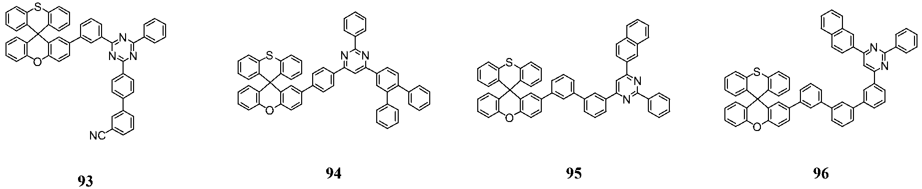

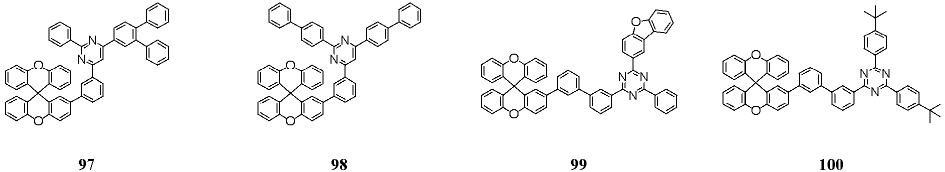

As used herein, "the number of nuclear atoms" means the number of ring atoms constituting a ring structure, and the nuclear atoms may mean carbon or a heteroatom selected from the group consisting of N, O, S and Se. For example, the number of nuclear atoms of pyridine means 6 including 5 C and 1 N constituting a pyridine ring.

As used herein, "alkyl" refers to a monovalent substituent derived from a linear or branched chain saturated hydrocarbon having 1 to 40 carbon atoms. Examples of such alkyl may include, but not limited to, methyl, ethyl, propyl, isobutyl, sec-butyl, pentyl, iso-amyl, hexyl or the like.

As used herein, "alkenyl" refers to a monovalent substituent derived from a C₂ to C₄₀ linear or branched chain unsaturated hydrocarbon, having at least one carbon-carbon double bond. Examples of such alkenyl may include, but not limited to, vinyl, allyl, isopropenyl, 2-butenyl or the like.

As used herein, "alkynyl" refers to a monovalent substituent derived from a C₂ to C₄₀ linear or branched chain unsaturated hydrocarbon, having at least one carbon-carbon triple bond. Examples of such alkynyl may include, but not limited to, ethynyl, 2-propynyl or the like.

As used herein, "aryl" refers to a monovalent substituent derived from a C₆ to C₄₀ aromatic hydrocarbon having a structure with a single ring or two or more rings combined with each other. In addition, a form in which two or more rings are pendant (e.g., simply attached) to or fused with each other may also be included. Examples of such aryl may include, but not limited to, phenyl, naphthyl, phenanthryl, anthryl or the like.

As used herein, "heteroaryl" refers to a monovalent substituent derived from a monoheterocyclic or polyheterocyclic aromatic hydrocarbon having 5 to 40 nuclear atoms. In such an embodiment, one or more carbons in the ring, preferably one to three carbons, are substituted with a heteroatom such as N, O, S or Se. In addition, a form in which two or more rings are pendant to or fused with each other may be included and a form fused with an aryl group may be included. Examples of such heteroaryl may include, but not limited to, a 6-membered monocyclic ring such as pyridyl, pyrazinyl, pyrimidinyl, pyridazinyl and triazinyl; a polycyclic ring such as phenoxathienyl, indolizinyl, indolyl, purinyl, quinolyl, benzothiazole, and carbazolyl; 2-furanyl; N-imidazolyl; 2-isoxazolyl; 2-pyridinyl; 2-pyrimidinyl or the like.

As used herein, "aryloxy" is a monovalent substituent represented by RO-, where R refers to a C₅ to C₄₀ aryl. Examples of such aryloxy may include, but not limited to, phenyloxy, naphthyloxy, diphenyloxy or the like.

As used herein, "alkyloxy" refers to a monovalent substituent represented by R'O-, where R' refers to a C₁ to C₄₀ alkyl. Such alkyloxy may include a linear, branched or cyclic structure. Examples of such alkyloxy may include, but not limited to, methoxy, ethoxy, n-propoxy, 1-propoxy, t-butoxy, n-butoxy, pentoxy or the like.

As used herein, "arylamine" refers to amine substituted with a C₆ to C₄₀ aryl.

As used herein, "cycloalkyl" refers to a monovalent substituent derived from a C₃ to C₄₀ monocyclic or polycyclic non-aromatic hydrocarbon. Examples of such cycloalkyl may include, but not limited to, cyclopropyl, cyclopentyl, cyclohexyl, norbornyl, adamantine or the like.

As used herein, "heterocycloalkyl" refers to a monovalent substituent derived from a non-aromatic hydrocarbon having 3 to 40 nuclear atoms, where one or more carbons in the ring, preferably one to three carbons, are substituted with a heteroatom such as N, S or Se. Examples of such heterocycloalkyl may include, but not limited to, morpholine, piperazine or the like.

As used herein, "alkylsilyl" refers to silyl substituted with a C₁ to C₄₀ alkyl, and "arylsilyl" refers to silyl substituted with a C₃ to C₄₀ aryl.

As used herein, the term "fused ring (e.g., condensed ring)" refers to a condensed aliphatic ring, a condensed aromatic ring, a condensed heteroaliphatic ring, a condensed heteroaromatic ring, or a combination thereof.

### <Electron transport layer material>

The present invention provides an electron transport layer including the compound represented by Chemical Formula 1.

The electron transport layer (ETL) serves to move electrons injected from a cathode to an adjacent layer, specifically a light emitting layer.

The compound represented by Chemical Formula 1 may be used alone as an electron transport layer (ETL) material, or may be used in combination with an electron transport layer material known in the art. It may preferably be used alone.

The electron transport layer material that may be used in combination with the compound of Chemical Formula 1 may include an electron transport material commonly known in the art. Nonlimiting examples of applicable electron transport materials may include oxazole-based compounds, isoxazole-based compounds, triazole-based compounds, isothiazole-based compounds, oxadiazole-based compounds, thiadiazole-based compounds, perylene-based compounds, aluminum complexes (e.g., tris(8-quinolinolato)-aluminium (Alq₃), BAlq, SAlq, Almq₃, gallium complexes (e.g., Gaq'20Piv, Gaq'20Ac, 2(Gaq'2)), etc. These may be used alone or two or more types thereof may be used in combination.

In the present invention, when the compound of Chemical Formula 1 and the electron transport layer material are used in combination, a mixing ratio thereof is not particularly limited, and may be appropriately adjusted within a range known in the art.

### <Auxiliary electron transport layer material>

In addition, the present invention provides an auxiliary electron transport layer including the compound represented by Chemical Formula 1.

The auxiliary electron transport layer is disposed between the light emitting layer and the electron transport layer and serves to substantially prevent diffusion of excitons or holes generated in the light emitting layer into the electron transport layer.

The compound represented by Chemical Formula 1 may be used alone as an auxiliary electron transport layer material, or may be combined with an electron transport layer material known in the art. It may preferably be used alone.

The auxiliary electron transport layer material that may be used in combination with the compound of Chemical Formula 1 includes an electron transport material commonly known in the art. For example, the auxiliary electron transport layer may include an oxadiazole derivative, a triazole derivative, a phenanthroline derivative (e.g., BCP), a heterocyclic derivative containing nitrogen, and the like.

In the present invention, when the compound of Chemical Formula 1 and the auxiliary electron transport layer material are used in combination, a mixing ratio thereof is not particularly limited, and may be appropriately adjusted within a range known in the art.

### <Organic electroluminescent device>

Another aspect of the present invention is directed to an organic electroluminescent device ("organic EL element") including the compound represented by Chemical Formula 1.

More specifically, the organic EL device according to the present invention includes an anode (e.g., a positive electrode), a cathode (e.g., a negative electrode), and one or more organic layers disposed between the anode and the cathode, and at least one of the one or more organic layers includes the compound represented by Chemical Formula 1. In such an embodiment, the compound may be used alone or in combination of two or more kinds thereof.

The one or more organic layers may be any one or more of a hole injection layer, a hole transport layer, a light emitting layer, a light emitting auxiliary layer, a lifespan improvement layer, an electron transport layer, an auxiliary electron transport layer and an electron injection layer, and at least one of the organic layers may include the compound represented by Chemical Formula 1. Specifically, the organic layer including the compound represented by Chemical Formula 1 may preferably be a light emitting layer, a light emitting auxiliary layer, an electron transport layer, an auxiliary electron transport layer and/or a lifespan improvement layer, and more specifically, it may be preferable to be an electron transport layer or an auxiliary electron transport layer.

The light emitting layer of the organic EL device of the present invention includes a host material and a dopant material, and may include the compound of Chemical Formula 1 as a host material. In addition, the light emitting layer of the present invention may include, as a host, other compounds known in the art rather than or in addition to the compound of Chemical Formula 1.

When the compound represented by Chemical Formula 1 is included as a material for the light emitting layer of the organic EL element, preferably as a blue, green, or red phosphorescent host material, since a bonding force between holes and electrons in the light emitting layer is increased, the efficiency (luminescence efficiency and power efficiency), lifespan, luminance, driving voltage, and the like of the organic EL device may be improved. Specifically, the compound represented by Chemical Formula 1 may be preferably included in an organic EL device as a green and/or red phosphorescent host, a fluorescent host, or a dopant material. In particular, the compound represented by Chemical Formula 1 of the present invention may preferably be a green phosphorescent exciplex N-type host material for a high-efficiency light emitting layer.

A structure of the organic EL device of the present invention is not particularly limited, and may be, for example, a structure in which a substrate, an anode, a hole injection layer, a hole transport layer, a light emitting auxiliary layer, a light emitting layer, an electron transport layer, and a cathode are sequentially stacked. In such a case, at least one of the hole injection layer, the hole transport layer, the light emitting auxiliary layer, the light emitting layer, the electron transport layer and the electron injection layer may include the compound represented by Chemical Formula 1, and preferably, the light emitting layer, more preferably a phosphorescent host, may include the compound represented by Chemical Formula 1. Meanwhile, an electron injection layer may be additionally stacked on the electron transport layer.

The organic EL device of the present invention may have a structure in which an insulating layer or an adhesive layer is inserted at an interface between the electrode and the organic layer.

The organic EL device of the present invention may be prepared using materials and methods known in the art to form organic layers and electrodes, except that one or more layers of the aforementioned organic layers include the compound represented by Chemical Formula 1.

The organic layer may be formed by a vacuum deposition method or a solution coating method. Examples of the solution coating method may include, but not limited to, spin coating, dip coating, doctor blading, inkjet printing, thermal transfer or the like.

The substrate used in preparation of the organic EL device of the present invention is not particularly limited, and nonlimiting examples thereof may include silicon wafers, quartz, glass plates, metal plates, plastic films, sheets or the like.

In addition, an anode material may use any anode material known in the art without limitation. Examples of the anode material may include, but not limited to, a metal such as vanadium, chromium, copper, zinc, and gold or an alloy thereof; metal oxides such as zinc oxide, indium oxide, indium tin oxide (ITO), or indium zinc oxide (IZO); combination of oxide with metal such as ZnO:Al or SnO₂:Sb; conductive polymers such as polythiophene, poly(3-methylthiophene), poly [3,4-(ethylene-1,2-dioxy) thiophene] (PEDT), polypyrrole or polyaniline; carbon black or the like.

In addition, a cathode material may use any cathode material known in the art without limitation. Examples of the cathode material may include, but not limited to, a metal such as magnesium, calcium, sodium, potassium, titanium, indium, yttrium, lithium, gadolinium, aluminum, silver, tin, or lead or an alloy thereof; a multi-layered material such as LiF/Al or LiO₂/Al or the like.

In addition, materials for the hole injection layer, the hole transport layer, the electron injection layer, and the electron transport layer are not particularly limited, and conventional materials known in the art may be used without limitation.

Hereinafter, the present invention will be described in detail through examples. However, the following examples are only to illustrate the present invention, and the present invention is not limited by the following examples.

### [Preparation Examples 1 to 12]

### [Preparation Example 1]

### Synthesis of Core 1

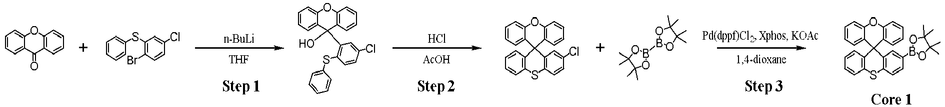

### <Step 1>

40.0 g(133.5 mmol) of (2-bromo-5-chlorophenyl) (phenyl)sulfane was added to 400 ml of THF, cooled to -78 °C, and then 56.1 ml(140.2 mmol) of n-BuLi(1.6M in n-hexane) was added dropwise thereto. A reaction solution obtained by cooling at -78 °C for 2 hours was added dropwise using a cannula into a reactor in which 26.2 g (133.5mmol) of 9H-xanthen-9-one was dissolved in 250 ml of THF at -78 °C. Then, the temperature was raised to room temperature, and then the reaction was performed for 4 hours. After the reaction was completed, 400 ml of H₂O and 400 ml of ethyl acetate were added to extract the organic layer, and then the organic layer was treated with anhydrous MgSO₄ and filtered. The filtered filtrate was concentrated under reduced pressure to obtain 50.1 g (yield 90%) of 9-(5-chloro-2-(phenylthio)phenyl)-9H-xanthen-9-ol.

### <Step 2>

50.1 g(120.2 mmol) of 9-(5-chloro-2-(phenylthio)phenyl)-9H-xanthen-9-ol was added to 350 ml of AcOH, and 50 ml of HCl was added thereto, and then the reaction was performed by stirring reflux for 4 hours. After the reaction was completed, the reaction product was cooled to room temperature, and then the resulting solid was filtered, and washed with 200 ml of H₂O. The solid was dissolved in dichloromethane, and then anhydrous treated with MgSO₄, and then filtered. The filtered filtrate was concentrated under reduced pressure, and subjected to column chromatography to obtain 41.8 g (yield 87.2%) of 2-chlorospiro[thioxanthene-9,9'-xanthene].

### <Step 3>

41.8 g(104.8 mmol) of 2-chlorospiro[thioxanthene-9,9'-xanthene], 31.9 g(125.7 mmol) of 4,4,4',4',5,5,5',5'-octamethyl-2,2'-bi(1,3,2-dioxaborolane), 2.3 g(3.1 mmol) of Pd(dppf)Cl₂, 30.8 g(314.4 mmol) of KOAc, and 3.0 g(6.3 mmol) of Xphos were added to 500 ml of 1,4-Dioxane, and heated to reflux for 12 hours. After the reaction was completed, the resulting mixture was extracted with dichloromethane, and MgSO₄ was added thereto to remove moisture, and then filtered. After filtering, the solvent of the organic layer was concentrated under reduced pressure, and purified by column chromatography using dichloromethane and hexane, and then solidified using methanol. The solid was filtered, washed with methanol, and dried in an oven to obtain 41.8 g (yield 81.4%) of Core 1.
Mass : [(M+H)⁺] : 491

### [Preparation Example 2]

### Synthesis of Core 2

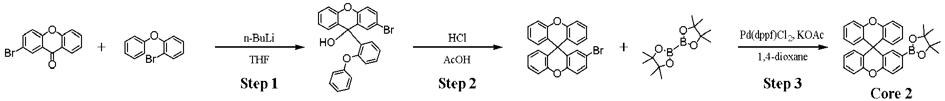

Except for using 50.0 g(200.7 mmol) of 1-bromo-2-phenoxybenzene and 55.2 g(200.7 mmol) of 2-bromo-9H-xanthen-9-one as starting materials, 56.8 g (two steps yield 66.2%) of 2-bromo-9,9'-spirobi[xanthene] was obtained by using the same method as in Step 1 and Step 2 of Preparation Example 1.

### <Step 3>

56.8g (132.9 mmol) of 2-bromo-9,9'-spirobi[xanthene], 40.5 g(159.5 mmol) of 4,4,4',4',5,5,5',5'-octamethyl-2,2'-bi(1,3,2-dioxaborolane), 2.9 g(4.0 mmol) of Pd(dppf)Cl₂, and 39.1 g(398.8 mmol) of KOAc were added to 600 ml of 1,4-Dioxane, and heated to reflux for 12 hours. After the reaction was completed, the resulting mixture was extracted with dichloromethane, and MgSO₄ was added thereto to remove moisture, and then filtered. After filtering, the solvent of the organic layer was concentrated under reduced pressure, and purified by column chromatography using dichloromethane and hexane, and then solidified using methanol. The solid was filtered, washed with methanol, and dried in an oven to obtain 47.9 g (yield 76.1%) of Core 2.
Mass : [(M+H)⁺] : 475

### [Preparation Example 3]

### Synthesis of Core 3

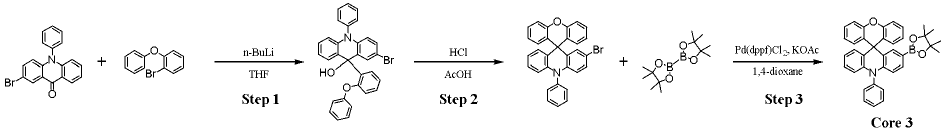

Except for using 50.0 g(200.7 mmol) of 1-bromo-2-phenoxybenzene and 70.3 g(200.7 mmol) of 2-bromo-10-phenylacridin-9(10H)-one, 53.5 g(three-step yield 48.5%) of Core 3 was obtained by using the same method as in Step 1 and Step 2 of Preparation Example 1 and Step 3 of Preparation Example 2.
Mass : [(M+H)⁺] : 550

### [Preparation Example 4]

### Synthesis of Core 4

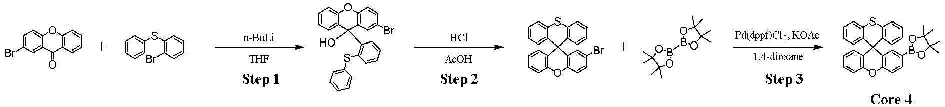

Except for using 50.0 g(188.6 mmol) of (2-bromophenyl) (phenyl)sulfane, and 51.9 g(188.6 mmol) of 2-bromo-9H-xanthen-9-one as starting materials, 38.0 g(three-step yield 41.1%) of Core 4 was obtained by using the same method as in Step 1 and Step 2 of Preparation Example 1 and Step 3 of Preparation Example 2.
Mass : [(M+H)⁺] : 491

### [Preparation Example 5]

### Synthesis of Core 5

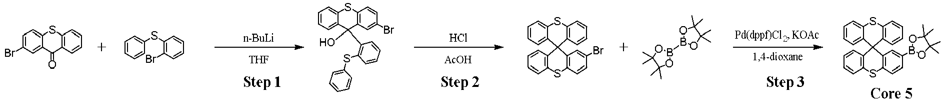

Except for using 50.0 g(188.6 mmol) of (2-bromophenyl) (phenyl)sulfane and 54.9 g(188.6 mmol) of 2-bromo-9H-thioxanthen-9-one as starting materials, 46.0 g(three-step yield 48.2%) of Core 5 was obtained by using the same method as in Step 1 and Step 2 of Preparation Example 1 and Step 3 of Preparation Example 2.
Mass : [(M+H)⁺] : 507

### [Preparation Example 6]

### Synthesis of Core 6

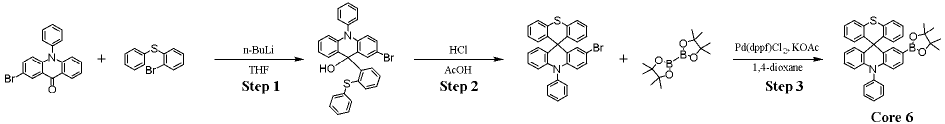

Except for using 50.0 g(188.6 mmol) of (2-bromophenyl) (phenyl)sulfane and 66.0 g(188.6 mmol) of 2-bromo-10-phenylacridin-9(10H)-one as starting materials, 41.5 g (three-step yield 38.9%) of Core 6 was obtained by using the same method as in Step 1 and Step 2 of Preparation Example 1 and Step 3 of Preparation Example 2.
Mass : [(M+H)⁺] : 566

### [Preparation Example 7]

### Synthesis of Core 7

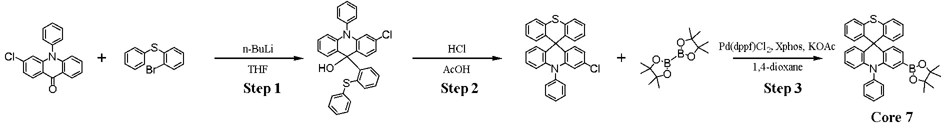

Except for using 50.0 g(188.6 mmol) of (2-bromophenyl) (phenyl)sulfane and 57.6 g(188.6 mmol) of 3-chloro-10-phenylacridin-9(10H)-one as starting materials, 37.6 g(three-step yield 35.3%) of Core 7 was obtained by using the same method as in Step 1 and Step 2 of Preparation Example 1 and Step 3 of Preparation Example 2.
Mass : [(M+H)⁺] : 566

### [Preparation Example 8]

### Synthesis of Core 8

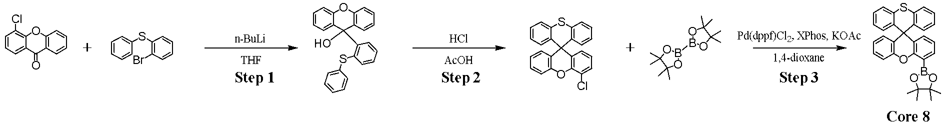

Except for using 50.0 g(188.6 mmol) of (2-bromophenyl) (phenyl)sulfane and 43.5 g(188.6 mmol) of 4-chloro-9H-xanthen-9-one as starting materials, 44.0 g(three-step yield 47.6%) of Core 8 was obtained by using the same method as in Step 1, Step 2 and Step 3 of Preparation Example 1.
Mass : [(M+H)⁺] : 491

### [Preparation Example 9]

### Synthesis of Core 9

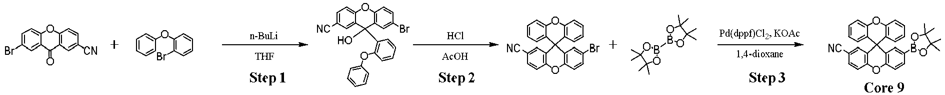

Except for using 50.0 g(200.7 mmol) of 1-bromo-2-phenoxybenzene and 60.2 g(200.7 mmol) of 7-bromo-9-oxo-9H-xanthene-2-carbonitrile as starting materials, 28.3 g(three-step yield 28.2%) of Core 9 was obtained by using the same method as in Step 1 and Step 2 of Preparation Example 1 and Step 3 of Preparation Example 2.
Mass : [(M+H)⁺] : 500

### [Preparation Example 10]

### Synthesis of Core 10

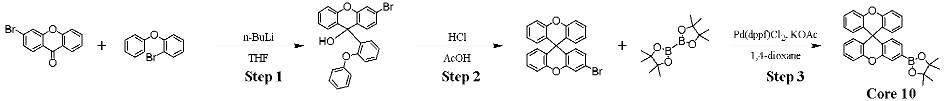

Except for using 50.0 g(200.7 mmol) of 1-bromo-2-phenoxybenzene and 55.2 g(200.7 mmol) of 3-bromo-9H-xanthen-9-one as starting materials, 40.1 g(three-step yield 42.1%) of Core 10 was obtained by using the same method as in Step 1 and Step 2 of Preparation Example 1 and Step 3 of Preparation Example 2.
Mass : [(M+H)⁺] : 475

### [Preparation Example 11]

### Synthesis of Core 11

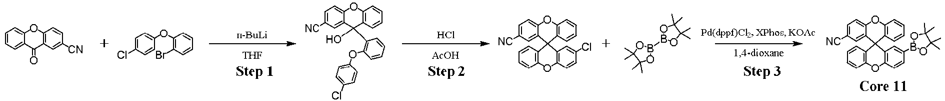

Except for using 50.0 g(176.3 mmol) of 1-bromo-2-(4-chlorophenoxy)benzene and 39.0 g(176.3 mmol) of 9-oxo-9H-xanthene-2-carbonitrile as starting materials, 27.5 g(three-step yield 31.2%) (three-step yield 47.6%) of Core 11 was obtained by using the same method as in Step 1, Step 2 and Step 3 of Preparation Example 1.
Mass : [(M+H)⁺] : 500

### [Preparation Example 12]

### Synthesis of Core 12

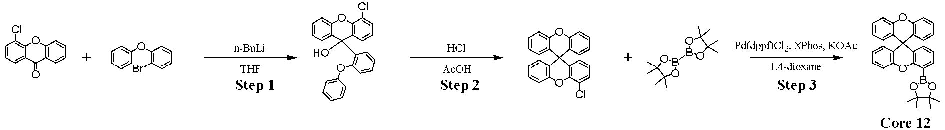

Except for using 50.0 g(200.7 mmol) of 1-bromo-2-phenoxybenzene and 46.3 g(200.7 mmol) of 4-chloro-9H-xanthen-9-one as starting materials, 41.8 g(three-step yield 43.9%) of Core 12 was obtained by using the same method as in Step 1, Step 2 and Step 3 of Preparation Example 1.
Mass : [(M+H)⁺] : 475

### [Synthesis Examples 1 to 21]

### [Synthesis Example 1] Synthesis of Compound 2

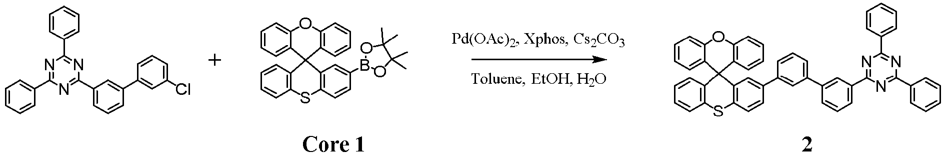

10.0 g(leq, 23.8 mmol) of 2-(3'-chloro-[1,1'-biphenyl]-3-yl)-4,6-diphenyl-1,3,5-triazine, 12.3 g(1.05eq, 25.0 mmol) of Core 1 of the Preparation Example 1, 0.2 g(0.03eq, 0.7 mmol) of Pd(OAc)₂, 23.3 g(3.0eq, 93.0 mmol) of Cs₂CO₃, and 0.7 g(0.06eq, 1.4 mmol) of Xphos were added to 200 ml of Toluene, 50 ml of EtOH, and 50 ml of H₂O, and the resulting mixture was heated to reflux for 4 hours. After the reaction was completed, the resulting mixture was extracted with dichloromethane, MgSO₄ was added thereto, and filtered. After removing the solvent of the filtered organic layer, the resulting mixture was subjected to column chromatography using dichloromethane and hexane, and then recrystallized with toluene acetone to obtain 13.9 g (yield 77.9 %) of Compound 2.
Mass : [(M+H)⁺] : 749

### [Synthesis Example 2] Synthesis of Compound 5

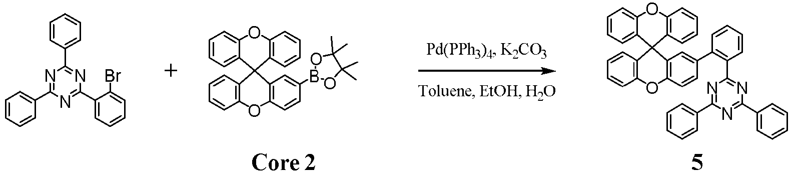

10.0 g(leq, 25.8 mmol) of 2-(2-bromophenyl)-4,6-diphenyl-1,3,5-triazine, 12.8 g(1.05eq, 27.0 mmol) of Core 2 of the Preparation Example 2, 0.9 g(0.03eq, 0.8 mmol) of Pd(Pph₃)₄, and 10.7 g(3.0eq, 77.3 mmol) of K₂CO₃ were added to 200 ml of Toluene, 50 ml of EtOH and 50 ml of H₂O, and the resulting mixture was heated to reflux for 4 hours. After the reaction was completed, the resulting mixture was extracted with dichloromethane, MgSO₄ was added thereto, and filtered. After removing the solvent of the filtered organic layer, the resulting mixture was subjected to column chromatography using dichloromethane and hexane, and then recrystallized with toluene acetone to obtain 13.9 g (yield 82.5 %) of Compound 5.
Mass : [(M+H)⁺] : 657

### [Synthesis Example 3] Synthesis of Compound 6

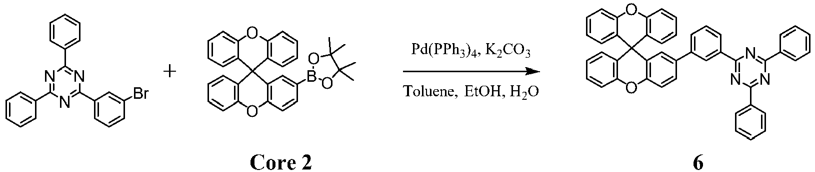

Except for using 10.0 g(25.8 mmol) of 2-(3-bromophenyl)-4,6-diphenyl-1,3,5-triazine as starting material, 15.0 g(yield 88.9%) of Compound 6 was obtained by using the same equivalents of regents and the same method as in Synthesis Example 2.
Mass : [(M+H)⁺] : 657

### [Synthesis Example 4] Synthesis of Compound 9

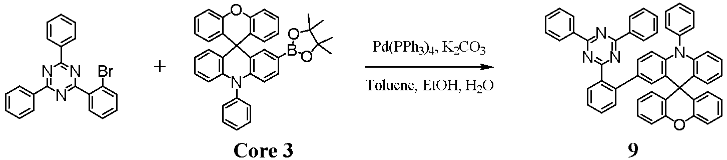

Except for using 10.0 g(leq, 25.8 mmol) of 2-(2-bromophenyl)-4,6-diphenyl-1,3,5-triazine and 14.9 g(1.05eq, 27.0 mmol) of Core 3 as starting material, 13.6 g(yield 72.3%) of Compound 9 was obtained by using the same equivalents of regents and the same method as in Synthesis Example 2.
Mass : [(M+H)⁺] : 732

### [Synthesis Example 5] Synthesis of Compound 16

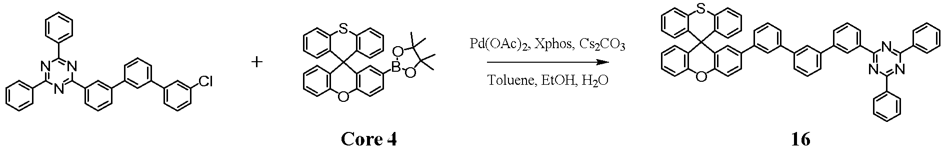

Except for using 10.0 g(leq, 20.2 mmol) of 2-(3''-chloro-[1,1':3',1"-terphenyl]-3-yl)-4,6-diphenyl-1,3,5-triazine and 10.4 g(1.05eq, 21.2 mmol) of Core 4 as starting material, 13.7 g(yield 82.3%) of Compound 16 was obtained by using the same equivalents of regents and the same method as in Synthesis Example 1.
Mass : [(M+H)⁺] : 825

### [Synthesis Example 6] Synthesis of Compound 22

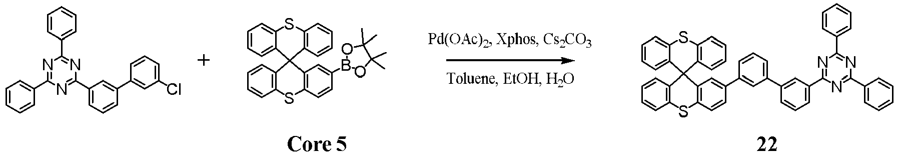

Except for using 10.0 g(1eq, 23.8 mmol) of 2-(3'-chloro-[1,1'-biphenyl]-3-yl)-4,6-diphenyl-1,3,5-triazine and 12.7 g(1.05eq, 25.0 mmol) of Core 5 as starting material, 13.2 g(yield 72.5%) of Compound 22 was obtained by using the same equivalents of regents and the same method as in Synthesis Example 1.
Mass : [(M+H)⁺] : 765

### [Synthesis Example 7] Synthesis of Compound 32

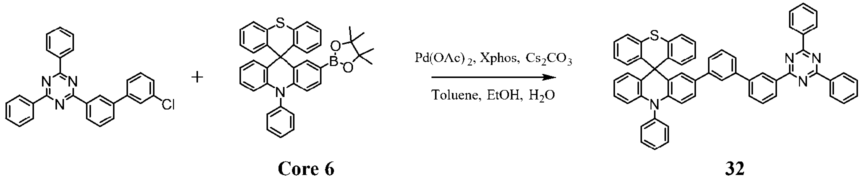

Except for using 10.0 g(leq, 23.8 mmol) of 2-(3'-chloro-[1,1'-biphenyl]-3-yl)-4,6-diphenyl-1,3,5-triazine and 14.1 g(1.05eq, 25.0 mmol) of Core 6 as starting material, 12.2 g(yield 62.1%) of Compound 32 was obtained by using the same equivalents of regents and the same method as in Synthesis Example 1.
Mass : [(M+H)⁺] : 824

### [Synthesis Example 8] Synthesis of Compound 45

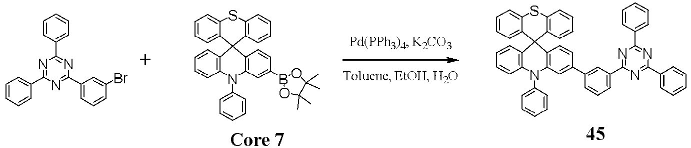

Except for using 10.0 g(1eq, 25.8 mmol) of 2-(3-bromophenyl)-4,6-diphenyl-1,3,5-triazine and 15.3 g(1.05eq, 27.0 mmol) of Core 7 as starting material, 13.5 g(yield 70.3%) of Compound 45 was obtained by using the same equivalents of regents and the same method as in Synthesis Example 2.
Mass : [(M+H)⁺] : 748

### [Synthesis Example 9] Synthesis of Compound 46

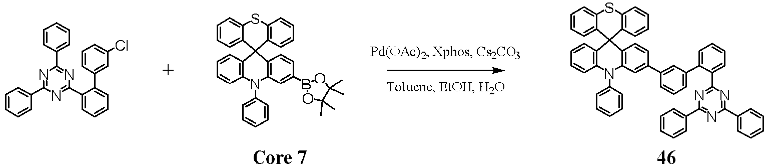

Except for using 10.0 g(leq, 23.8 mmol) of 2-(3'-chloro-[1,1'-biphenyl]-2-yl)-4,6-diphenyl-1,3,5-triazine and 14.1 g(1.05eq, 25.0 mmol) of Core 7 as starting material, 10.8 g(yield 55.1%) of Compound 46 was obtained by using the same equivalents of regents and the same method as in Synthesis Example 1.
Mass : [(M+H)⁺] : 824

### [Synthesis Example 10] Synthesis of Compound 54

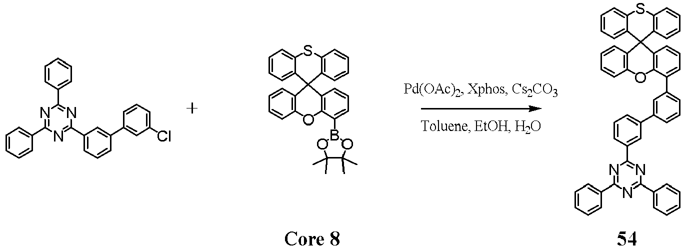

Except for using 10.0 g(1eq, 23.8 mmol) of 2-(3'-chloro-[1,1'-biphenyl]-2-yl)-4,6-diphenyl-1,3,5-triazine and 12.3 g(1.05eq, 25.0 mmol) of Core 8 as starting material, 10.8 g(yield 60.7%) of Compound 54 was obtained by using the same equivalents of regents and the same method as in Synthesis Example 1.
Mass : [(M+H)⁺] : 749

### [Synthesis Example 11] Synthesis of Compound 71

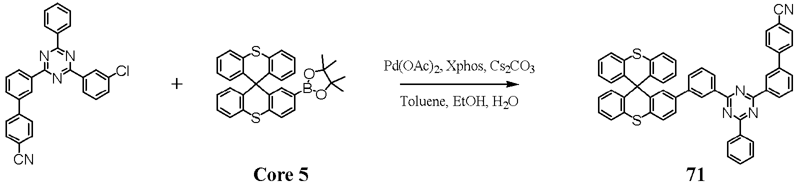

Except for using 10.0 g(1eq, 22.5 mmol) of 3'-(4-(3-chlorophenyl)-6-phenyl-1,3,5-triazin-2-yl)-[1,1'-biphenyl]-4-carbonitrile and 11.9 g(1.05eq, 23.6 mmol) of Core 5 as starting material, 12.9 g(yield 73.2%) of Compound 71 was obtained by using the same equivalents of regents and the same method as in Synthesis Example 1.
Mass : [(M+H)⁺] : 790

### [Synthesis Example 12] Synthesis of Compound 73

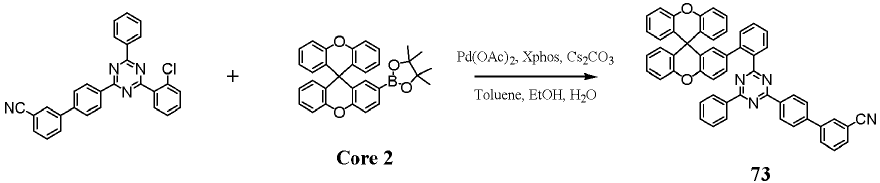

Except for using 10.0 g(leq, 22.5 mmol) of 4'-(4-(2-chlorophenyl)-6-phenyl-1,3,5-triazin-2-yl)-[1,1'-biphenyl]-3-carbonitrile and 11.2 g(1.05eq, 23.6 mmol) of Core 2 as starting material, 10.9 g(yield 63.8%) of Compound 73 was obtained by using the same equivalents of regents and the same method as in Synthesis Example 1.
Mass : [(M+H)⁺] : 758

### [Synthesis Example 13] Synthesis of Compound 74

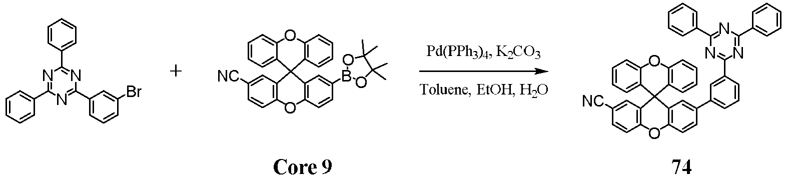

Except for using 10.0 g(1eq, 25.8 mmol) of 2-(3-bromophenyl)-4,6-diphenyl-1,3,5-triazine and 13.5 g(1.05eq, 27.0 mmol) of Core 9 as starting material, 12.6 g(yield 72.1%) of Compound 74 was obtained by using the same equivalents of regents and the same method as in Synthesis Example 2.
Mass : [(M+H)⁺] : 682

### [Synthesis Example 14] Synthesis of Compound 76

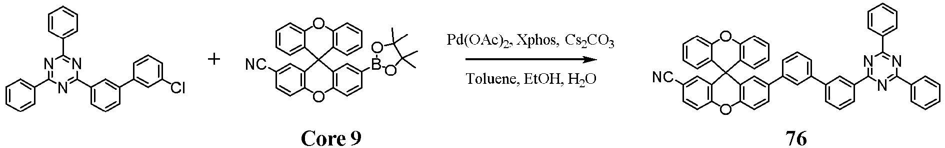

Except for using 10.0 g(1eq, 23.8 mmol) of 2-(3'-chloro-[1,1'-biphenyl]-3-yl)-4,6-diphenyl-1,3,5-triazine and 12.5 g(1.05eq, 25.0 mmol) of Core 9 as starting material, 11.4 g(yield 63.3%) of Compound 76 was obtained by using the same equivalents of regents and the same method as in Synthesis Example 1.
Mass : [(M+H)⁺] : 758

### [Synthesis Example 15] Synthesis of Compound 77

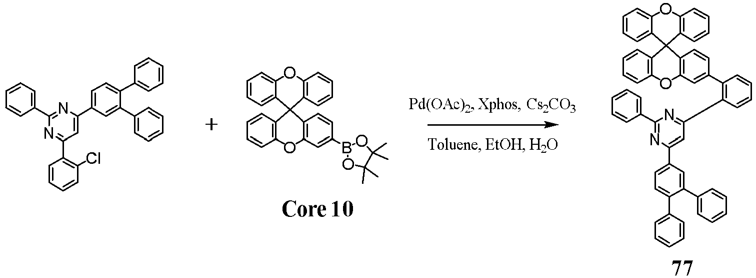

Except for using 10.0 g(leq, 20.2 mmol) of 4-([1,1':2',1"-terphenyl]-4'-yl)-6-(2-chlorophenyl)-2-phenylpyrimidine and 10.1 g(1.05eq, 21.2 mmol) of Core 10 as starting material, 8.9 g(yield 54.2%) of Compound 77 was obtained by using the same equivalents of regents and the same method as in Synthesis Example 1.
Mass : [(M+H)⁺] : 808

### [Synthesis Example 16] Synthesis of Compound 78

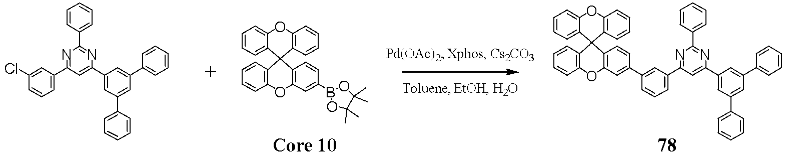

Except for using 10.0 g(leq, 20.2 mmol) of 4-([1,1':3',1"-terphenyl]-5'-yl)-6-(3-chlorophenyl)-2-phenylpyrimidine and 10.1 g(1.05eq, 21.2 mmol) of Core 10 as starting material, 13.5 g(yield 82.9%) of Compound 78 was obtained by using the same equivalents of regents and the same method as in Synthesis Example 1.
Mass : [(M+H)⁺] : 808

### [Synthesis Example 17] Synthesis of Compound 84

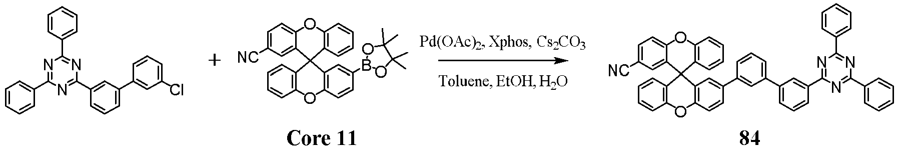

Except for using 10.0 g(leq, 23.8 mmol) of 2-(3'-chloro-[1,1'-biphenyl]-3-yl)-4,6-diphenyl-1,3,5-triazine and 12.49 g(1.05eq, 25.0 mmol) of Core 11 as starting material, 13.0 g(yield 72.1%) of Compound 84 was obtained by using the same equivalents of regents and the same method as in Synthesis Example 1.
Mass : [(M+H)⁺] : 758

### [Synthesis Example 18] Synthesis of Compound 90

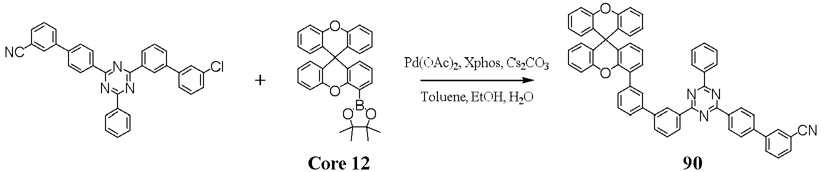

Except for using 10.0 g(leq, 19.2 mmol) of 4'-(4-(3'-chloro-[1,1'-biphenyl]-3-yl)-6-phenyl-1,3,5-triazin-2-yl)-[1,1'-biphenyl]-3-carbonitrile and 9.6 g(1.05eq, 20.2 mmol) of Core 12 as starting material, 7.8 g(yield 48.8%) of Compound 90 was obtained by using the same equivalents of regents and the same method as in Synthesis Example 1.
Mass : [(M+H)⁺] : 834

### [Synthesis Example 19] Synthesis of Compound 92

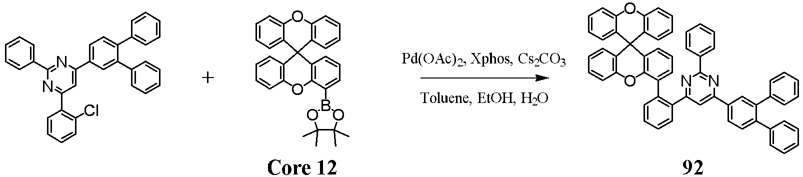

Except for using 10.0 g(leq, 20.2 mmol) of 4-([1,1':2',1"-terphenyl]-4'-yl)-6-(2-chlorophenyl)-2-phenylpyrimidine and 10.1 g(1.05eq, 21.2 mmol) of Core 12 as starting material, 8.3 g(yield 50.8%) of Compound 92 was obtained by using the same equivalents of regents and the same method as in Synthesis Example 1.
Mass : [(M+H)⁺] : 808

### [Synthesis Example 20] Synthesis of Compound 93

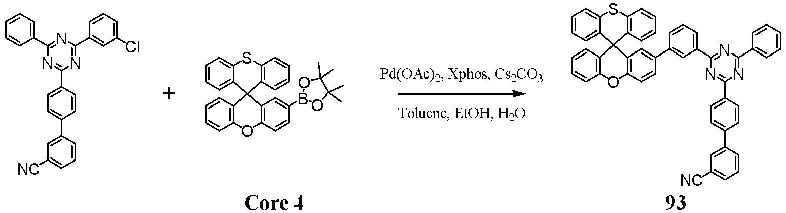

Except for using 10.0 g(1eq, 22.5 mmol) of 4'-(4-(3-chlorophenyl)-6-phenyl-1,3,5-triazin-2-yl)-[1,1'-biphenyl]-3-carbonitrile and 11.6 g(1.05eq, 23.6 mmol) of Core 4 as starting material, 10.6 g(yield 61.2%) of Compound 93 was obtained by using the same equivalents of regents and the same method as in Synthesis Example 1.
Mass : [(M+H)⁺] : 774

### [Synthesis Example 21] Synthesis of Compound 97

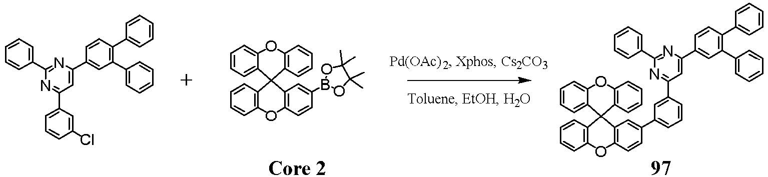

Except for using 10.0 g(leq, 20.2 mmol) of 4-([1,1':2',1"-terphenyl]-4'-yl)-6-(3-chlorophenyl)-2-phenylpyrimidine and 10.1 g(1.05eq, 21.2 mmol) of Core 2 as starting material, 14.2 g(yield 86.9%) of Compound 97 was obtained by using the same equivalents of regents and the same method as in Synthesis Example 1.
Mass : [(M+H)⁺] : 808

### [Examples 1 to 7] Manufacturing of Blue fluorescent organic EL devices

The compounds synthesized in the above synthesis examples were subjected to high-purity sublimation purification in a conventionally known method, and then blue organic EL devices were manufactured according to the following procedure.

A glass substrate thin-film-coated with indium tin oxide (ITO) to a thickness of 1,200 Å was washed with distilled water ultrasonically. After washing with distilled water was completed, the glass substrate was ultrasonically washed with a solvent, such as isopropyl alcohol, acetone and methanol, dried, transferred to a UV OZONE cleaner (Power sonic 405, Hwasin Tech), cleaned for 5 minutes using UV, and then transferred to a vacuum evaporator.

On the ITO transparent electrode prepared as above, HT-1 + 2% HAT-CN (100 Å) / HT-1 (1400 Å) / HT-2 (50 Å) / BH + 2% BD (200 Å) / ET-2 (50 Å) / each compounds 71 ~ 93 in Table 1 below : LiQ = 1 : 1 (300 Å) / LiF (10 Å ) / Al (1000 Å) were stacked in order to manufacture an organic EL device.

In such a case, the structures of HT-1, HAT-CN, HT-2, BH, BD, ET-1, ET-2 and LiQ are each as follows.

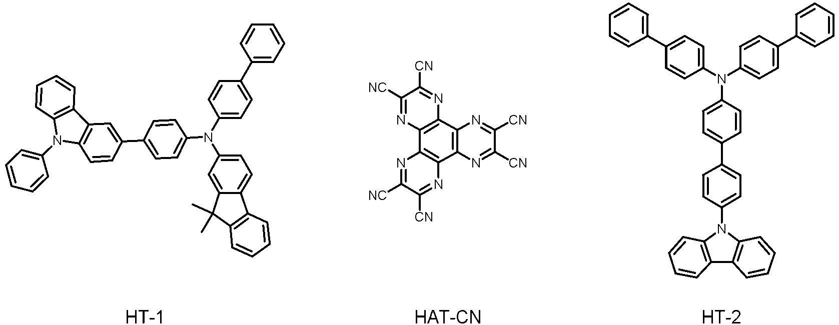

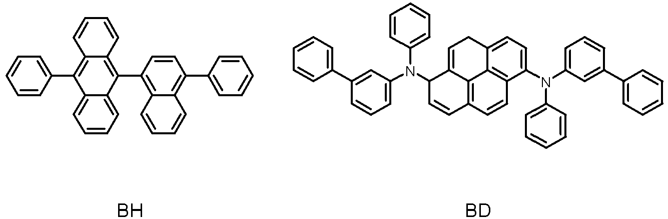

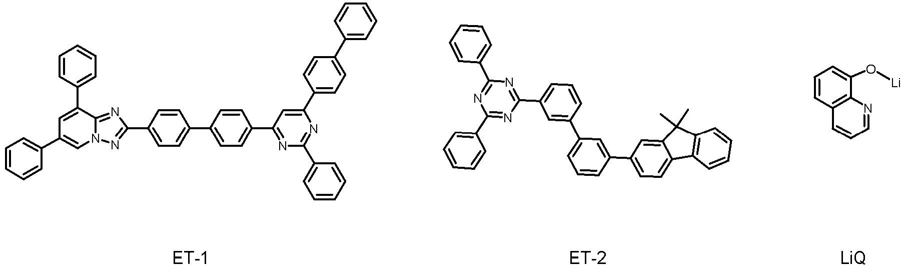

### [Comparative Example 1] Manufacturing of blue fluorescent organic EL device

A blue fluorescent organic EL device of Comparative Example 1 was manufactured in the same manner as in Example 1, except that Compound ET-1 was used instead of Compound 71 used as the electron transport layer material in Example 1.

### [Evaluation example 1]

For each of the blue organic EL devices manufactured in Examples (Ex.) 1 to 7 and Comparative Example (Comp. Ex.) 1, a driving voltage, a current efficiency at a current density of 10 mA/cm², and an emission peak were measured, and the results are shown in Table 1 below.

**[Table 1]**

| Sample | Electron transport layer | Driving voltage (V) | EL peak (nm) | Current efficiency (cd/A) |
|---|---|---|---|---|
| Ex. 1 | Compound 71 | 4.3 | 460 | 6.6 |
| Ex. 2 | Compound 73 | 4.6 | 461 | 6.7 |
| Ex. 3 | Compound 74 | 4.4 | 460 | 6.7 |
| Ex. 4 | Compound 76 | 4.5 | 461 | 6.5 |
| Ex. 5 | Compound 84 | 4.1 | 459 | 6.9 |
| Ex. 6 | Compound 90 | 4.2 | 458 | 6.5 |
| Ex. 7 | Compound 93 | 4.6 | 460 | 6.2 |
| Comp. Ex. 1 | ET-1 | 4.7 | 460 | 6.0 |

As shown in Table 1, the blue organic electroluminescent devices of Examples 1 to 7 using the compounds of the present invention as electron transport layer materials exhibited excellent performance in terms of the driving voltage, light emission peak, and current efficiency, compared with the blue organic electroluminescent devices of Comparative Example 1 in which the conventional compound ET-1 containing no spirobixanthene-based core was used as an electron transport layer material.

### [Examples 8 to 21] Manufacturing of Blue fluorescent organic EL devices

The compounds synthesized in the above synthesis examples were subjected to high-purity sublimation purification in a conventionally known method, and then blue organic EL devices were manufactured according to the following procedure.

A glass substrate thin-film-coated with indium tin oxide (ITO) to a thickness of 1,500 Å was washed with distilled water ultrasonically. After washing with distilled water was completed, the glass substrate was ultrasonically washed with a solvent, such as isopropyl alcohol, acetone and methanol, dried, transferred to a UV OZONE cleaner (Power sonic 405, Hwasin Tech), cleaned for 5 minutes using UV, and then transferred to a vacuum evaporator.

On the ITO transparent electrode prepared as above, HT-1 + 2% HAT-CN (100 Å) / HT-1 (1400 Å) / HT-2 (50 Å) / BH + 2% BD (200 Å) / each compound 2 ~ 97 in Table 2 below (50 Å) / ET-1 : LiQ = 1:1 (300 Å) / LiF (10 Å ) / Al (1000 Å) were stacked in order to manufacture an organic EL device.

### [Comparative Examples 2 to 5] Manufacturing of blue fluorescent organic EL device

A blue organic EL device of Comparative Examples 2 to 5 were each manufactured in the same manner as in Example 8, except that Compounds ET-2 to ET-5 were deposited at 50 Å instead of Compound 2 used as the auxiliary electron transport layer material.

In such a case, the structures of Compounds ET-2, ET-3, ET-4 and ET-5 used in Comparative Examples 2 to 5 are each as follows.

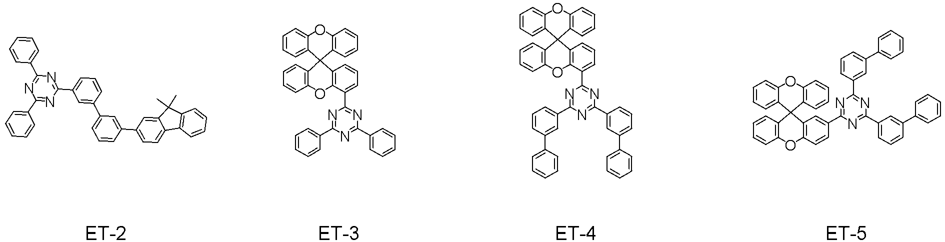

### [Evaluation example 2]

For each of the organic EL devices manufactured in Examples (Ex.) 8 to 21 and Comparative Examples (Comp. Ex.) 2 to 5, a driving voltage, a current efficiency at a current density of 10 mA/cm², and an emission peak were measured, and the results are shown in Table 2 below.

**[Table 2]**

| Sample | Electron transport layer | Driving voltage (V) | EL peak (nm) | Current efficiency (cd/A) |
|---|---|---|---|---|
| Ex. 8 | Compound 2 | 4.6 | 461 | 6.5 |
| Ex. 9 | Compound 5 | 4.0 | 460 | 7.2 |
| Ex. 10 | Compound 6 | 4.5 | 462 | 6.9 |
| Ex. 11 | Compound 9 | 4.3 | 460 | 7.0 |
| Ex. 12 | Compound 16 | 4.5 | 458 | 6.3 |
| Ex. 13 | Compound 22 | 4.6 | 460 | 6.4 |
| Ex. 14 | Compound 32 | 4.4 | 461 | 6.6 |
| Ex. 15 | Compound 45 | 4.3 | 460 | 6.7 |
| Ex. 16 | Compound 46 | 4.3 | 462 | 6.9 |
| Ex. 17 | Compound 54 | 4.6 | 459 | 6.7 |
| Ex. 18 | Compound 77 | 4.2 | 462 | 7.0 |
| Ex. 19 | Compound 78 | 3.9 | 460 | 7.3 |
| Ex. 20 | Compound 92 | 4.3 | 461 | 6.9 |
| Ex. 21 | Compound 97 | 4.1 | 459 | 7.1 |
| Comp. Ex. 2 | ET-2 | 4.7 | 460 | 6.3 |
| Comp. Ex. 3 | ET-3 | 4.8 | 461 | 5.3 |
| Comp. Ex. 4 | ET-4 | 4.6 | 460 | 5.2 |
| Comp. Ex. 5 | ET-5 | 4.9 | 462 | 5.3 |

As shown in Table 2, the blue organic electroluminescent devices of Examples 8 to 21 using the compounds of the present invention as auxiliary electron transport layer materials exhibited excellent performance in terms of current efficiency and the driving voltage compared to the blue organic electroluminescent devices of Comparative Examples 2 to 5, which respectively included the compound ET-2 that does not comprise a spirobixanthene-based core, or the compounds ET-3 to ET-5, in which the azine group is directly linked to the spirobixanthene-based core without a linker, as the electron transport auxiliary layer material.

## Claims

1. A compound represented by Chemical Formula 1: 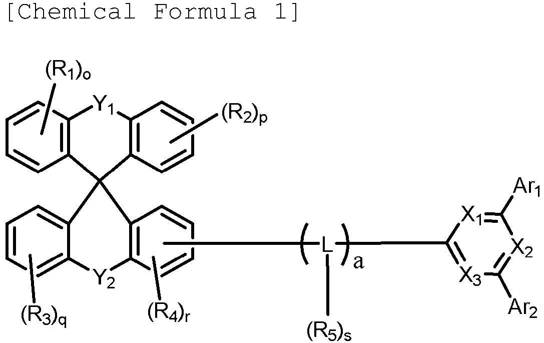
wherein in Chemical Formula 1,
X₁ to X₃ are the same as or different from each other and are each independently C(R₆) or N, provided that at least two of X₁ to X₃ are N,
Y₁ to Y₂ are the same as or different from each other and are each independently selected from the group consisting of O, S, NR₁₁,
Ar₁ and Ar₂ are the same as or different from each other and are each independently selected from the group consisting of a C₁-C₄₀ alkyl group, a C₂-C₄₀ alkenyl group, a C₂-C₄₀ alkynyl group, a C₃-C₄₀ cycloalkyl group, a heterocycloalkyl group having 3 to 40 ring atoms, a C₆-C₆₀ aryl group, a heteroaryl group having 5 to 60 ring atoms, a C₁-C₄₀ alkyloxy group, a C₆-C₆₀ aryloxy group, a C₃-C₄₀ alkylsilyl group, a C₆-C₆₀ arylsilyl group, a C₁-C₄₀ alkylboron group, a C₆-C₆₀ arylboron group, a C₆-C₆₀ arylphosphine group, a C₆-C₆₀ arylphosphine oxide group and a C₆-C₆₀ arylamine group, or combine with an adjacent group to form a fused ring;
R₁ to R₆ and R₁₁ are the same as or different from one another and are each independently selected from the group consisting of a hydrogen, a deuterium (D), a halogen, a cyano group, a nitro group, a C₁-C₄₀ alkyl group, a C₂-C₄₀ alkenyl group, a C₂-C₄₀ alkynyl group, a C₃-C₄₀ cycloalkyl group, a heterocycloalkyl group having 3 to 40 ring atoms, a C₆-C₆₀ aryl group, a heteroaryl group having 5 to 60 ring atoms, a C₁-C₄₀ alkyloxy group, a C₆-C₆₀ aryloxy group, a C₃-C₄₀ alkylsilyl group, a C₆-C₆₀ arylsilyl group, a C₁-C₄₀ alkylboron group, a C₆-C₆₀ arylboron group, a C₆-C₆₀ aryl phosphine group, a C₆-C₆₀ arylphosphine oxide group, and a C₆-C₆₀ arylamine group, or combine with an adjacent group to form a fused ring;
L is selected from the group consisting of a C₆ to C₁₈ arylene group and a heteroarylene group having 5 to 18 nuclear atoms,
a is an integer of 1 to 5, and when a is 2 or more, a plurality of L groups is the same as or different from each other,
o, p, q, and s are each independently an integer of 0 to 4, r is an integer of 0 to 3, and
the arylene group, the heteroarylene group of L; and the alkyl group, alkenyl group, alkynyl group, aryl group, heteroaryl group, aryloxy group, alkyloxy group, cycloalkyl group, heterocycloalkyl group, alkylsilyl group, arylsilyl group, alkylboron group, arylboron group, arylphosphine group, arylphosphine oxide group, and arylamine group of Ar₁ to Ar₂, R₁ to R₆ and R₁₁ may be each independently substituted with at least one substituent selected from the group consisting of a deuterium (D), a halogen, a cyano group, a nitro group, a C₁ to C₄₀ alkyl group, a C₂ to C₄₀ alkenyl group, a C₂ to C₄₀ alkynyl group, a C₃ to C₄₀ cycloalkyl group, a heterocycloalkyl group having 3 to 40 ring atoms, a C₆ to C₆₀ aryl group, a heteroaryl group having 5 to 60 ring atoms, a C₁ to C₄₀ alkyloxy group, a C₆ to C₆₀ aryloxy group, a C₁ to C₄₀ alkylsilyl group, a C₆ to C₆₀ arylsilyl group, a C₁ to C₄₀ alkylboron group, a C₆ to C₆₀ arylboron group, a C₆ to C₆₀ arylphosphine group, a C₆ to C₆₀ arylphosphine oxide group, and a C₆ to C₆₀ arylamine group, and wherein when the substituent is present in a plural number, they may be the same or different from each other.

2. The compound of Claim 1, wherein the Y₁ to Y₂ containing ring is selected from the group of substituents represented by the following structural formulas: 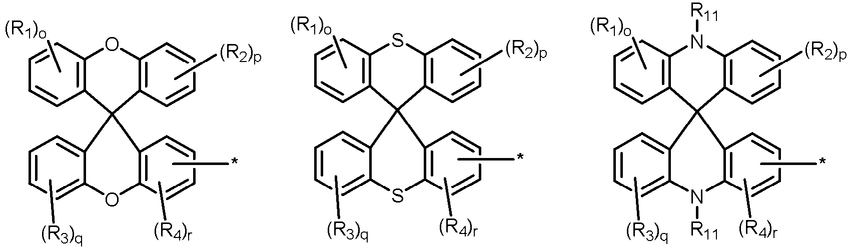 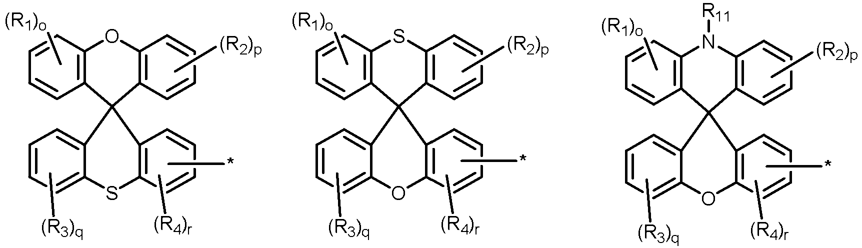 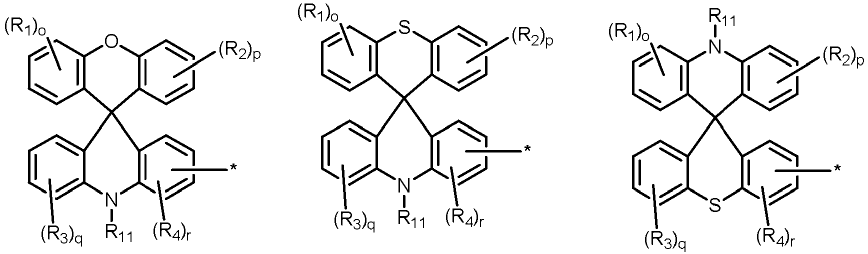
wherein the formulas,
* indicates a site where a bond with Chemical Formula 1 is made,
R₁₁ is selected from the group consisting of a hydrogen, a deuterium (D), a C₁-C₄₀ alkyl group, a C₆-C₆₀ aryl group, and a heteroaryl group having 5 to 60 ring atoms, and
R₁ to R₄, o, p, q and r are the same as defined in claim 1.

3. The compound of Claim 1, wherein the X containing moiety is selected from the group of substituents represented by the following structural formulas: 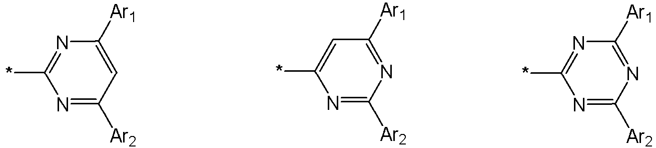 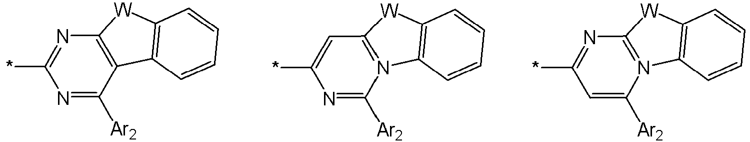 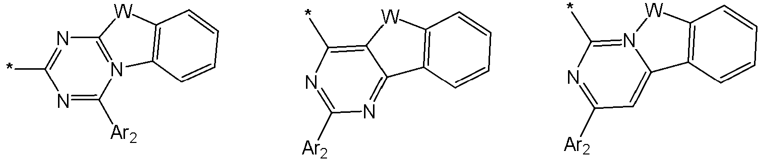 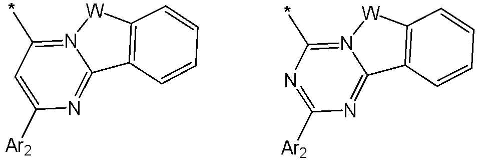
wherein the formulas,
* indicates a site where a bond with Chemical Formula 1 is made,
W is O or S, and
Ar₁ and Ar₂ are the same as defined in claim 1.

4. The compound of claim 1, wherein Ar₁ and Ar₂ are the same as or different from each other, and are each independently selected from the group consisting of a C₆ to C₆₀ aryl group and a heteroaryl group having 5 to 60 nuclear atoms.

5. The compound of claim 1, wherein Ar₁ and Ar₂ are each independently selected from the following structural formulas: 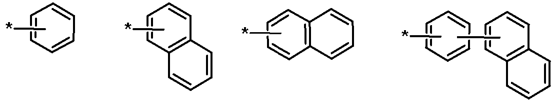 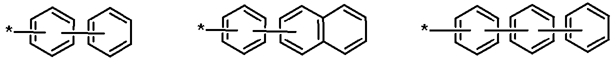 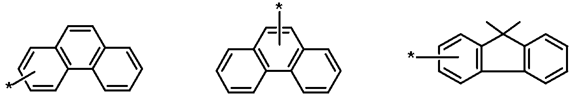 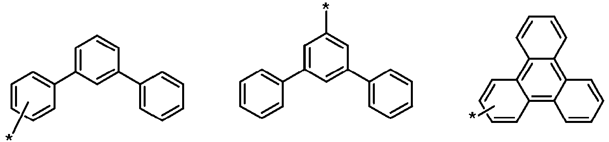 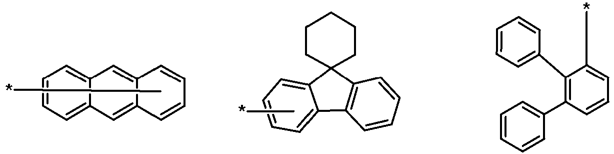 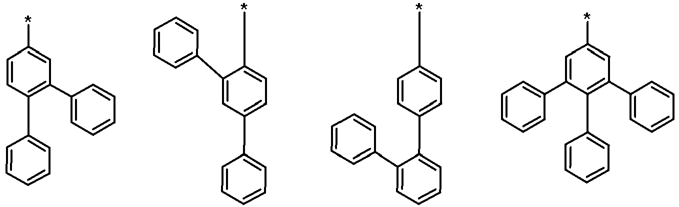 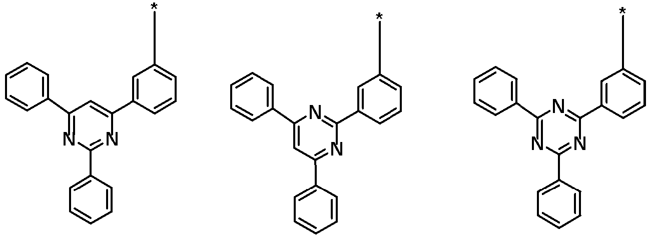 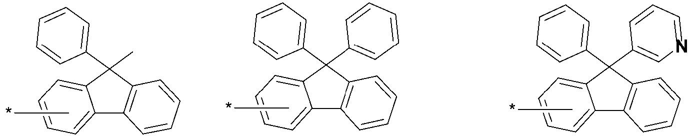 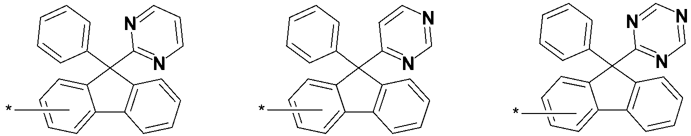 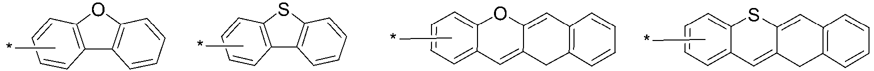 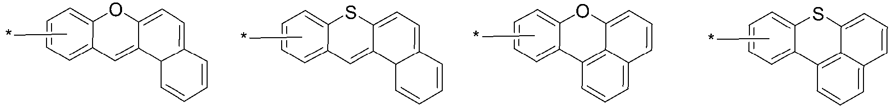 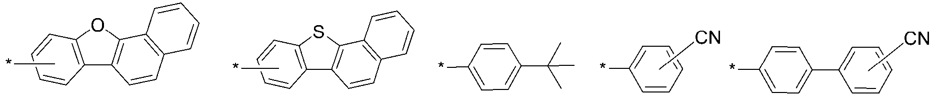
wherein the formulas,
* indicates a site where a bond with Chemical Formula 1 is made.

6. The compound of Claim 1, wherein L is a linker selected from the group of substituents represented by the following structural formulas: 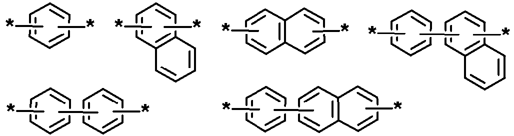 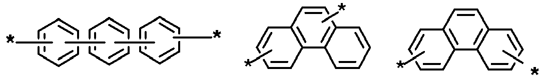 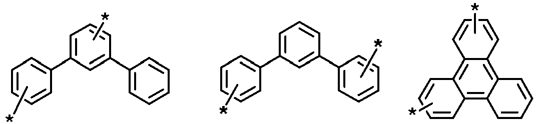 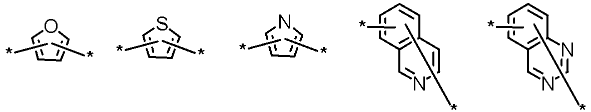 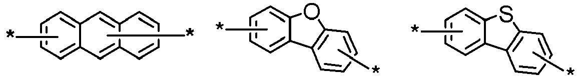
wherein the formulas,
* indicates a site where a bond with Chemical Formula 1 is made.

7. The compound of claim 1, wherein R₁ to R₅ are the same as or different from each other, and are each independently selected from the group consisting of a hydrogen, a deuterium (D), a cyano group, a C₁-C₄₀ alkyl group, a C₆-C₆₀ aryl group, and a heteroaryl group having 5 to 60 ring atoms, or combine with an adjacent group to form a fused ring.

8. The compound of claim 1, wherein the compound represented by Chemical Formula 1 is a compound represented by any one of Chemical Formula 2 to Chemical Formula 10 below: 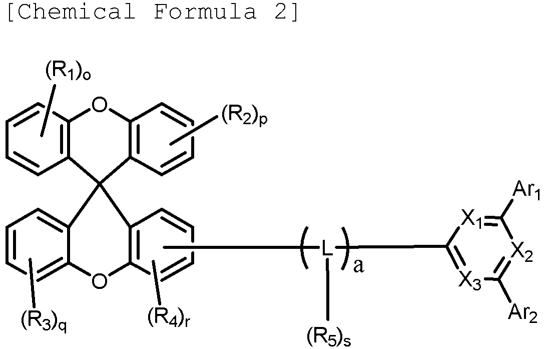 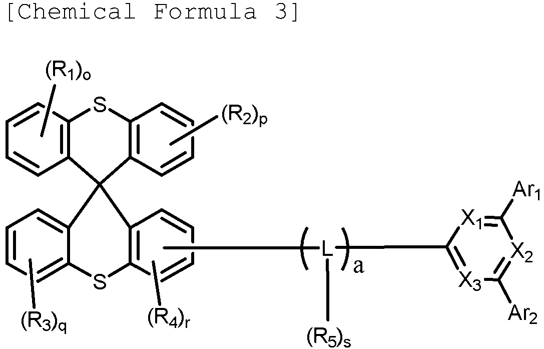 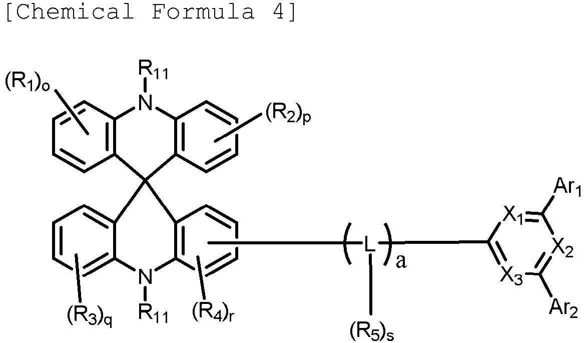 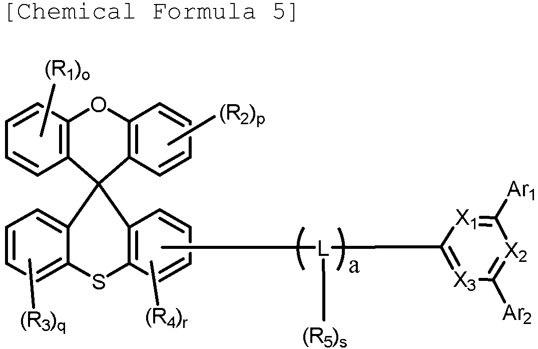 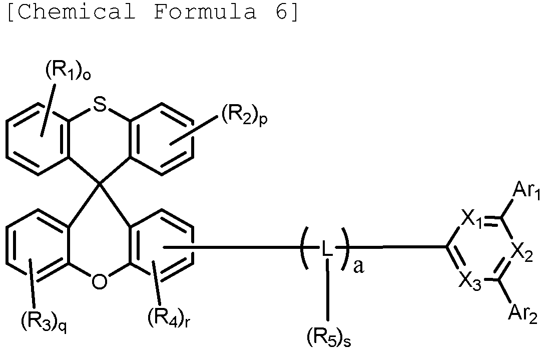 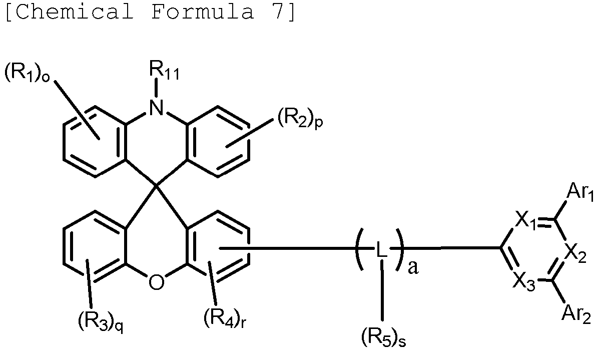 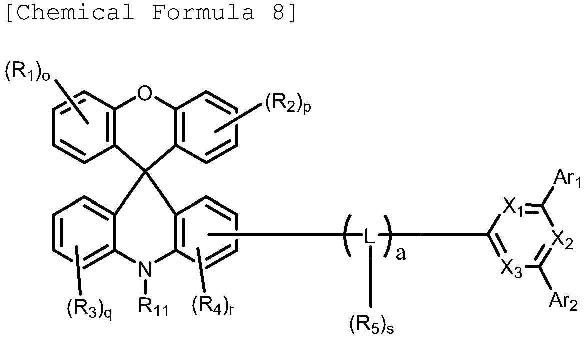 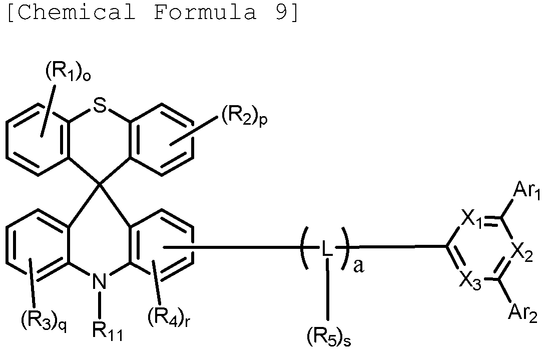 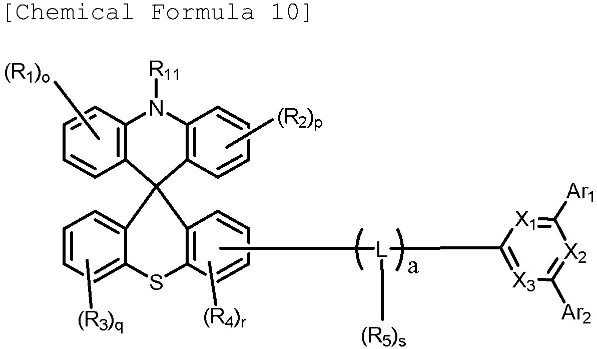
wherein the formulas,
X₁ to X₃, Ar₁ to Ar₂, L, R₁ to R₅, R₁₁, o, p, q, r, s and a are the same as defined in claim 1.

9. The compound of claim 1, wherein the compound represented by Chemical Formula 1 is a compound represented by any one of Chemical Formula 11 to Chemical Formula 29 below: 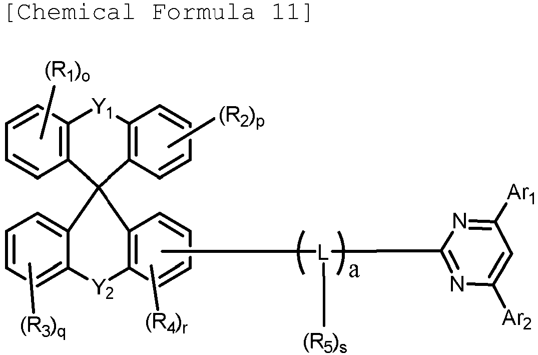 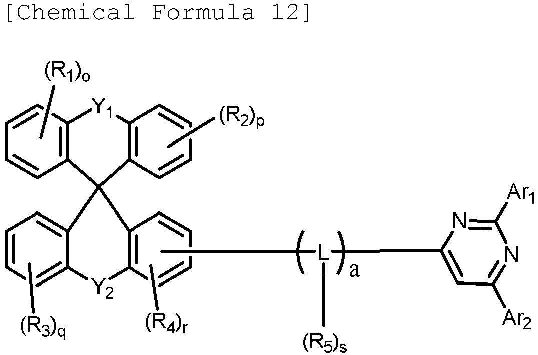 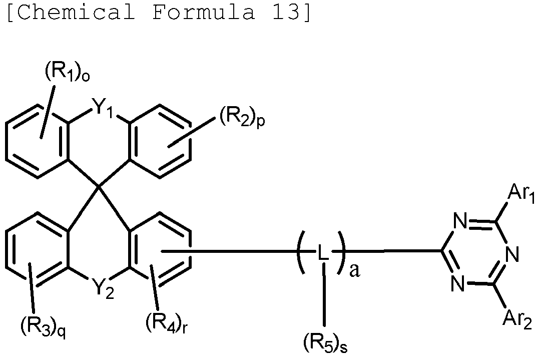 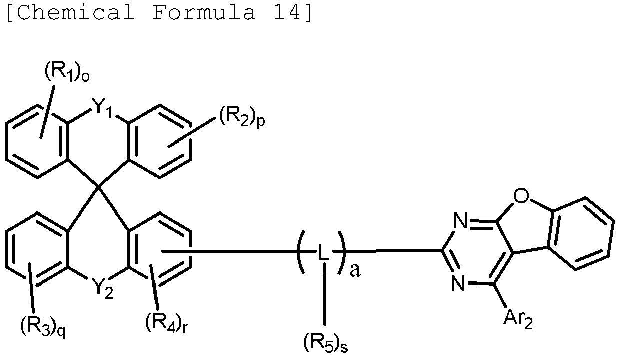 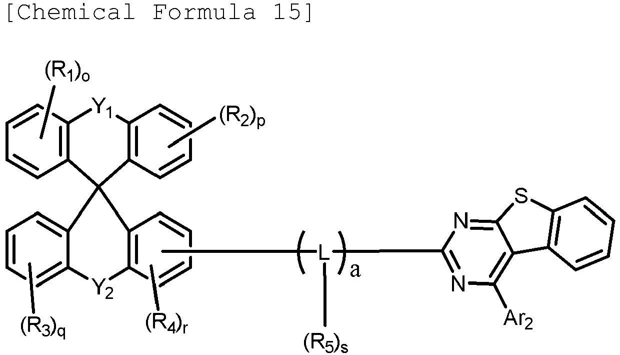 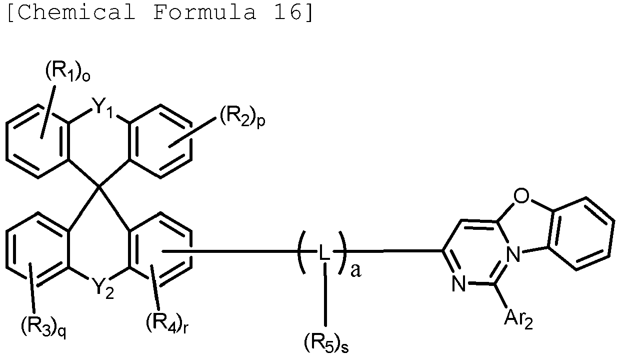 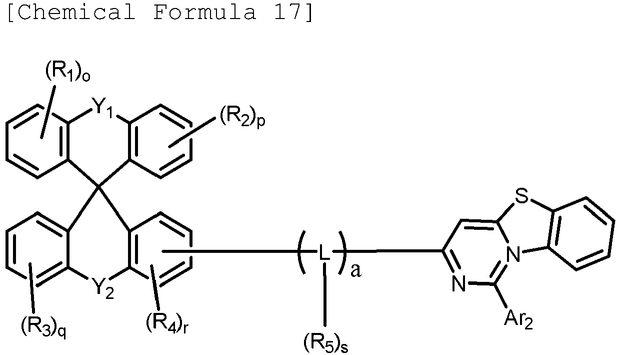 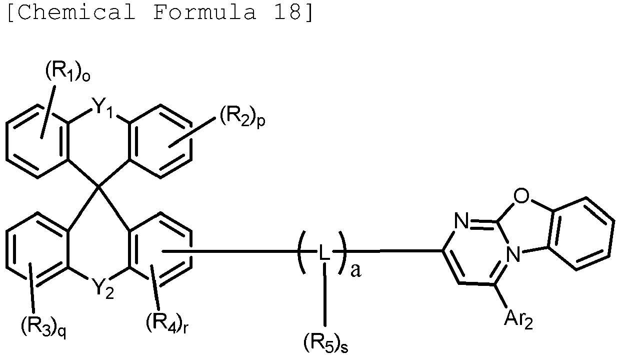 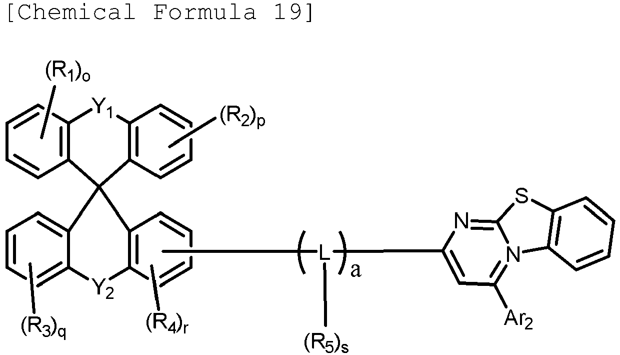 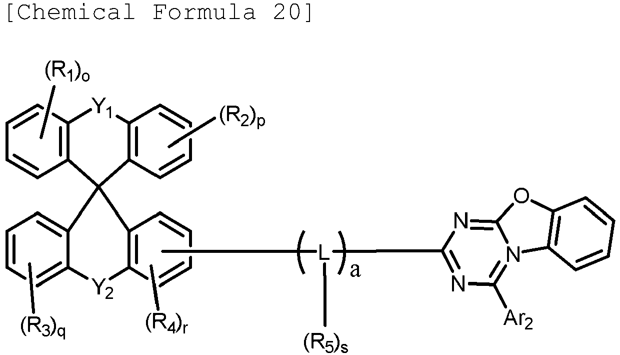 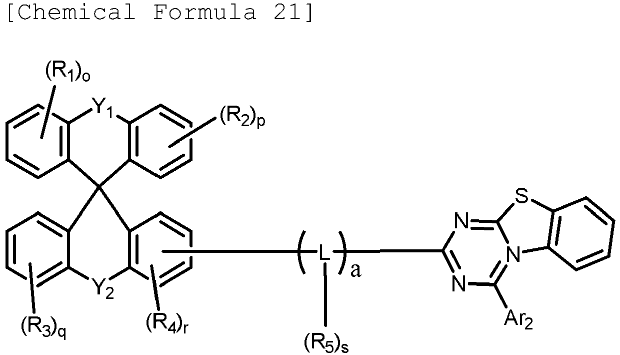 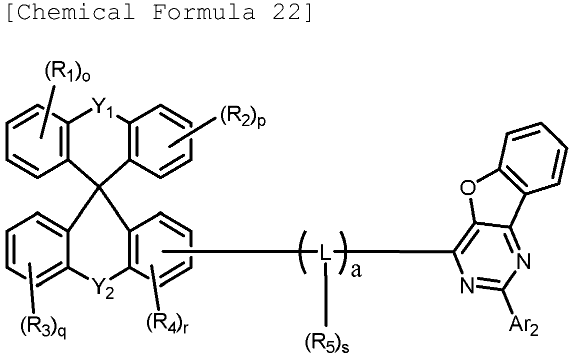 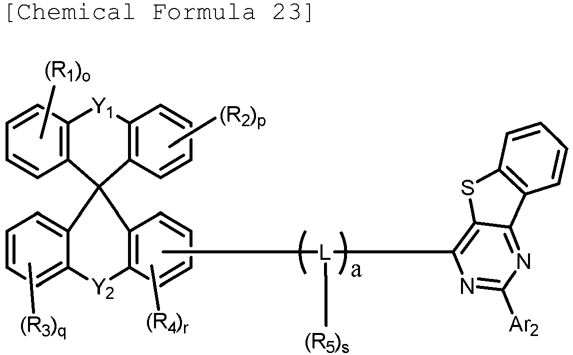 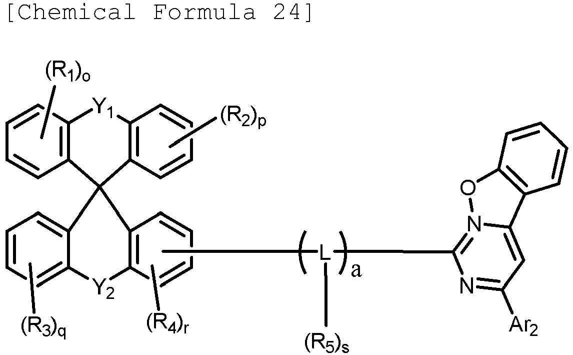 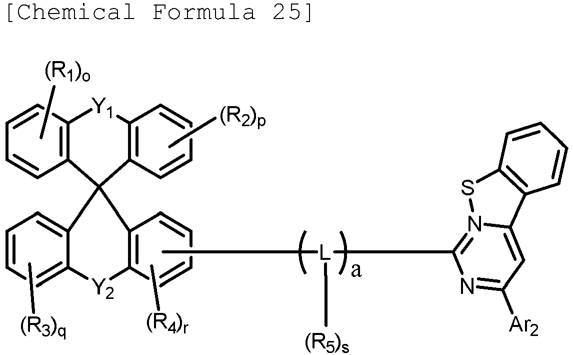 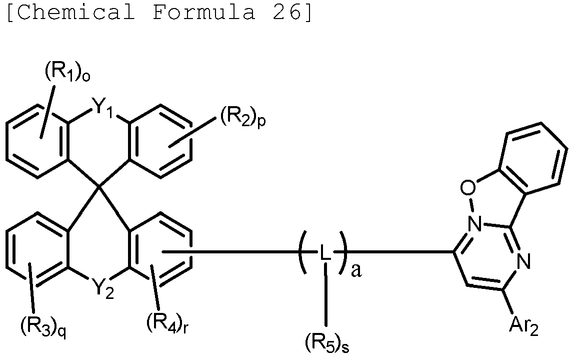 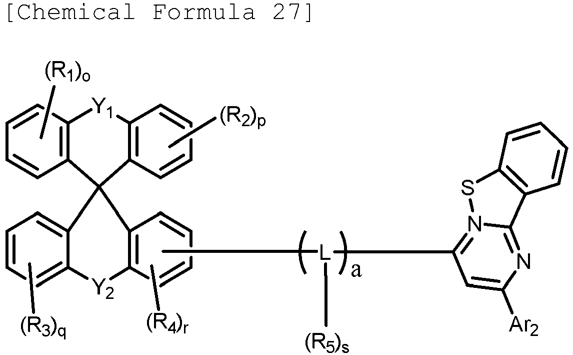 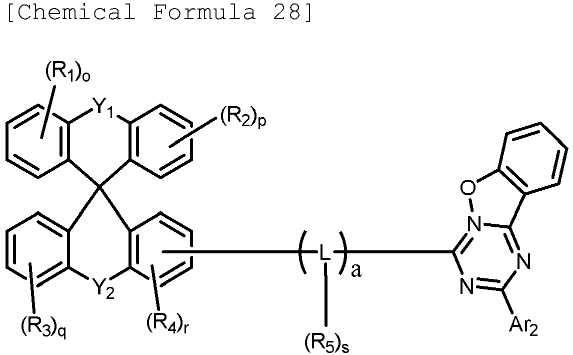 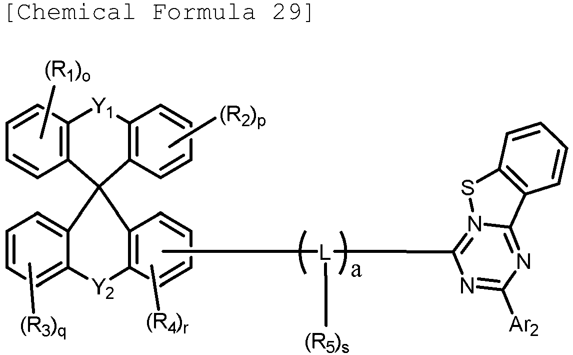
wherein the formulas,
Y₁ to Y₂, Ar₁ to Ar₂, L, R₁ to R₅, o, p, q, r, s and a are the same as defined in claim 1.

10. The compound of claim 1, wherein the compound represented by Chemical Formula 1 is a compound represented by any one of Chemical Formula 30 to Chemical Formula 37 below: 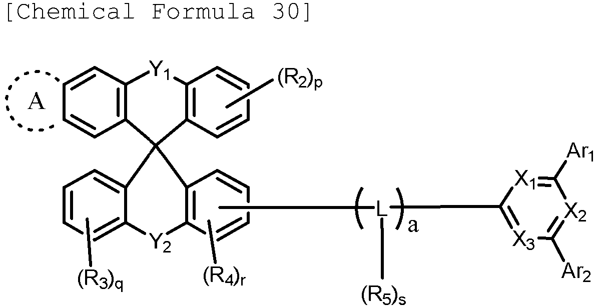 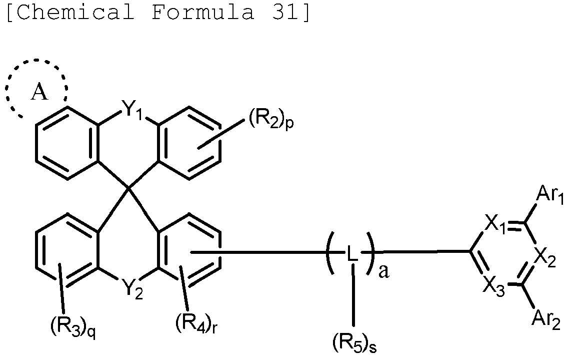 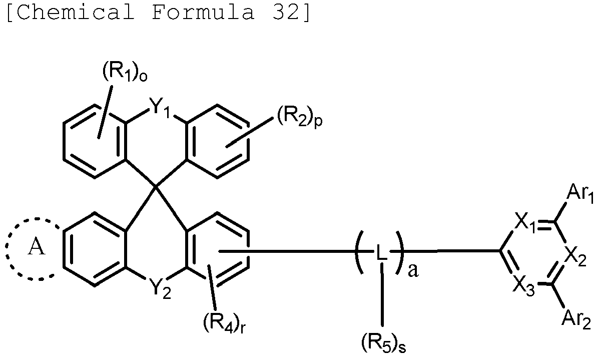 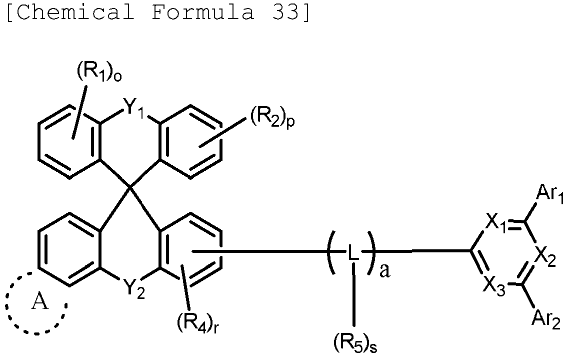 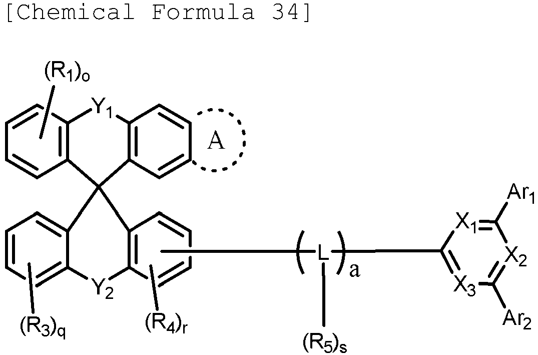 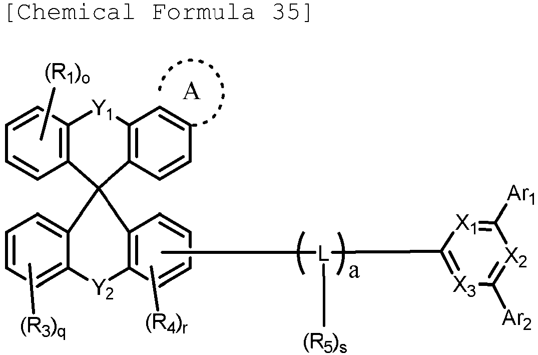 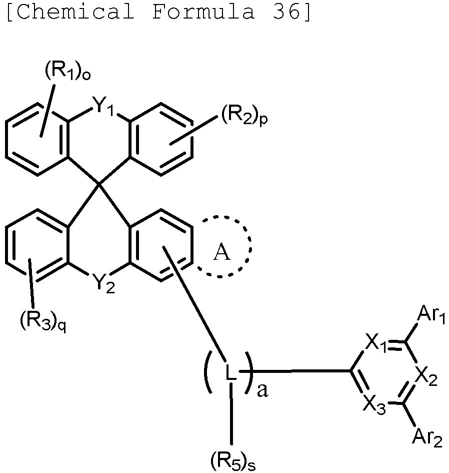 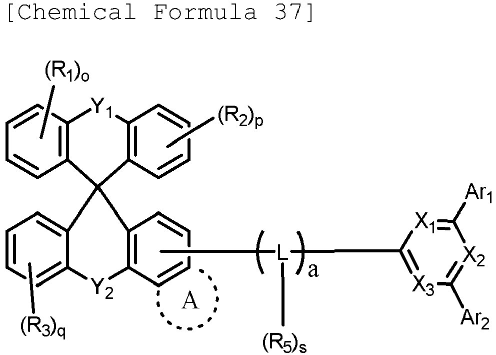
wherein the formulas,
Ring A is a monocyclic or polycyclic alicyclic ring, a monocyclic or polycyclic heteroalicyclic ring, a monocyclic or polycyclic aromatic ring, or a monocyclic or polycyclic heteroaromatic ring, and
X₁ to X₃, Y₁ to Y₂, Ar₁ to Ar₂, L, R₁ to R₅, o, p, q, r, s and a are the same as defined in claim 1.

11. The compound of claim 1, wherein the compound represented by Chemical Formula 1 is a compound represented by any one of Chemical Formula 38 to Chemical Formula 40 below: 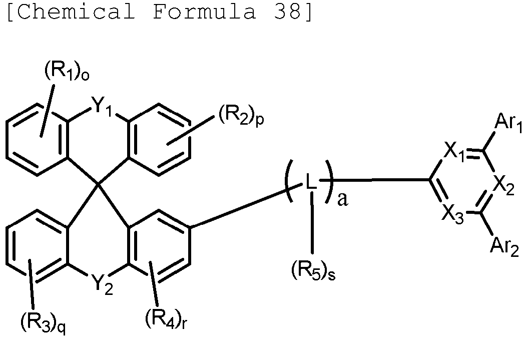 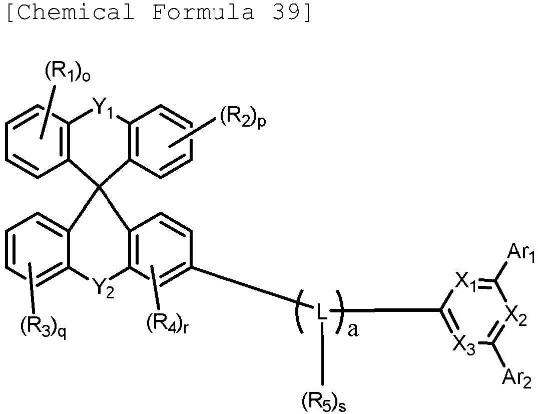 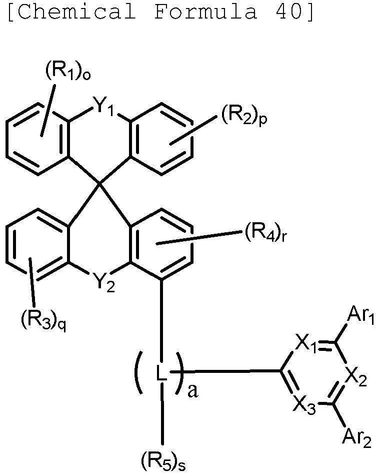
wherein the formulas,
X₁ to X₃, Y₁ to Y₂, Ar₁ to Ar₂, L, R₁ to R₅, o, p, q, r, s and a are the same as defined in claim 1.

12. The compound of claim 1, wherein the compound represented by Chemical Formula 1 is a compound represented by any one of Chemical Formula 41 to Chemical Formula 42 below: 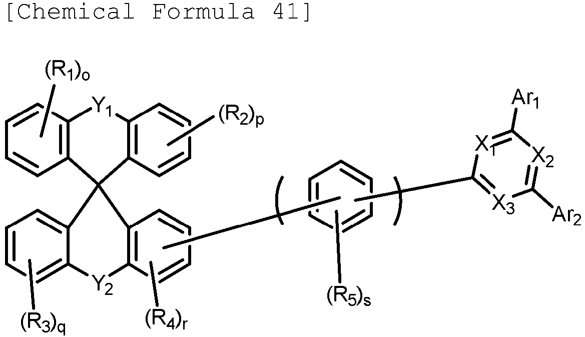 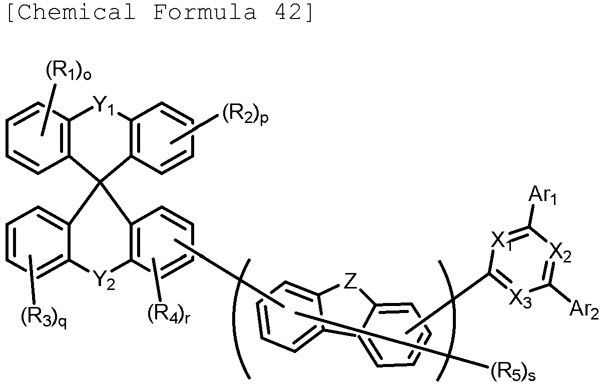
wherein the formulas,
Z is O or S, and
X₁ to X₃, Y₁ to Y₂, Ar₁ to Ar₂, R₁ to R₅, o, p, q, r, s and a are the same as defined in claim 1.

13. The compound of claim 1, wherein the compound represented by Chemical Formula 1 is selected from compounds represented by any one of the following Chemical Formulas 1 to 100.

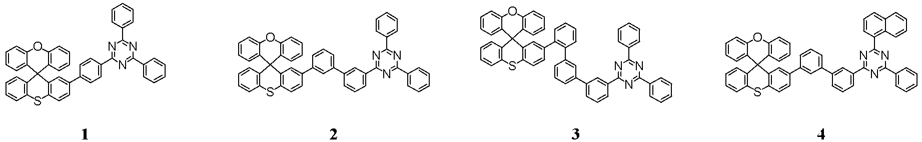

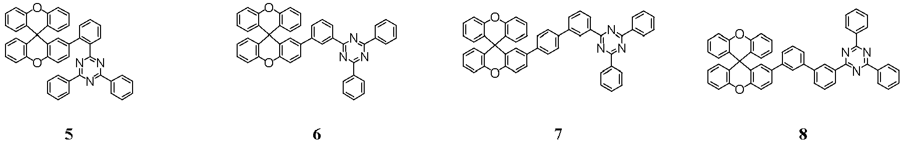

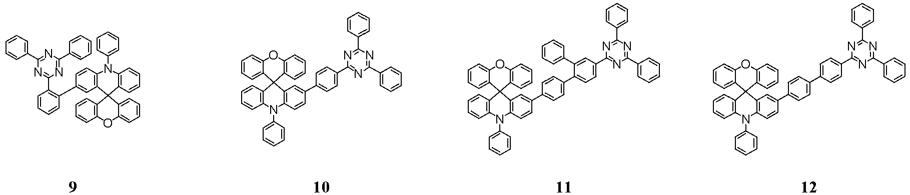

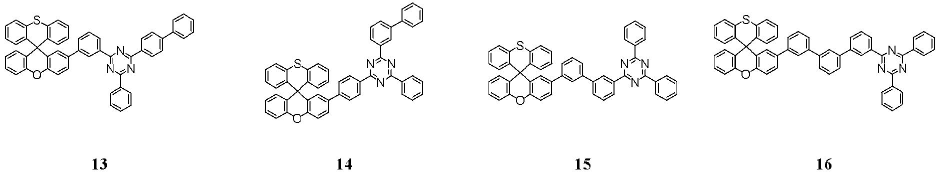

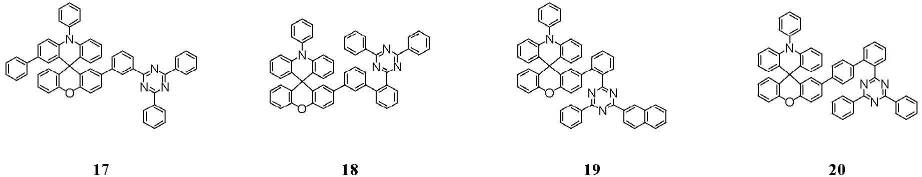

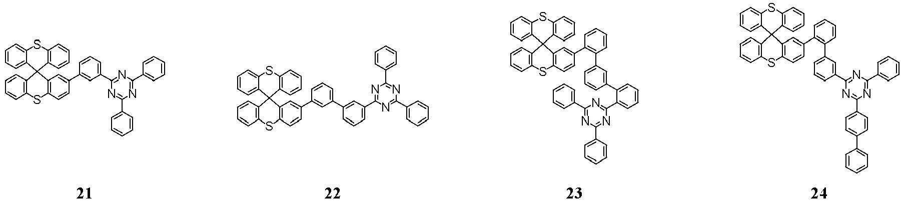

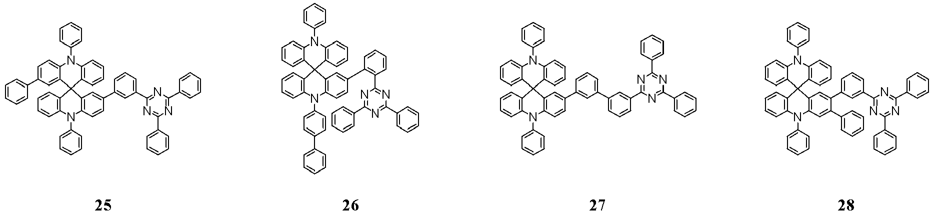

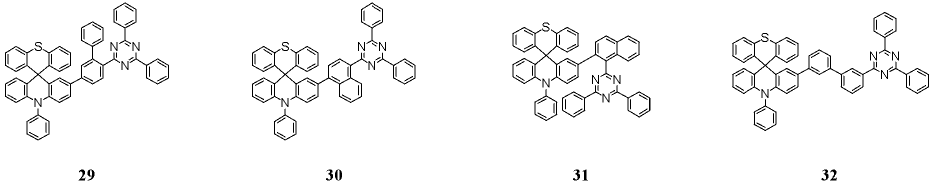

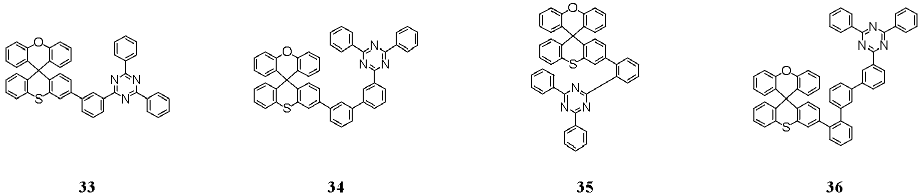

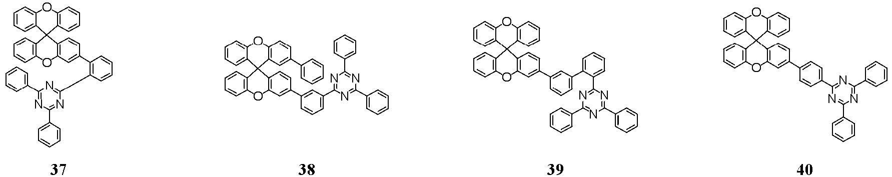

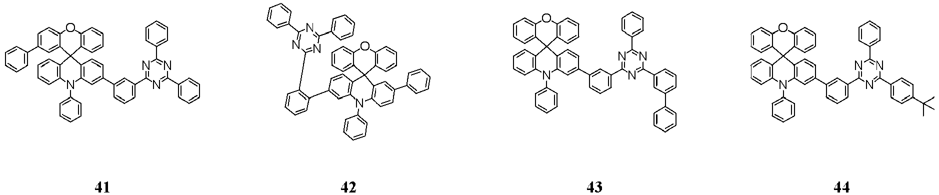

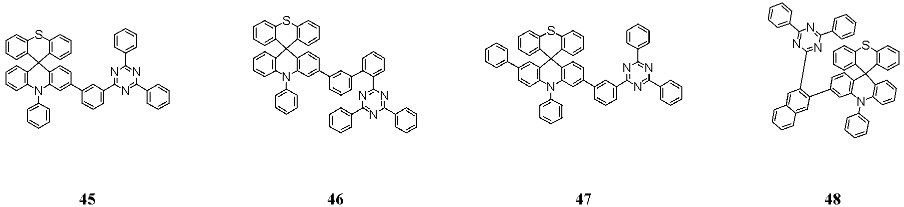

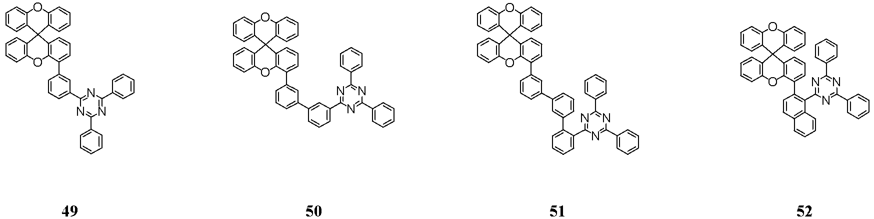

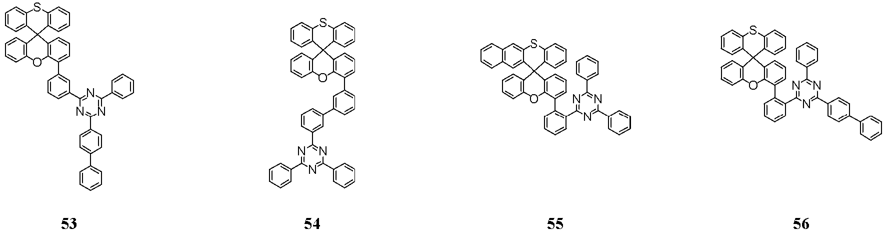

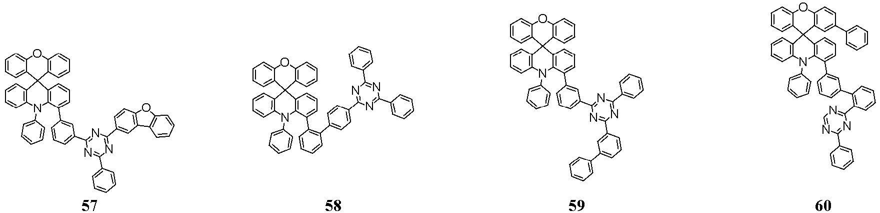

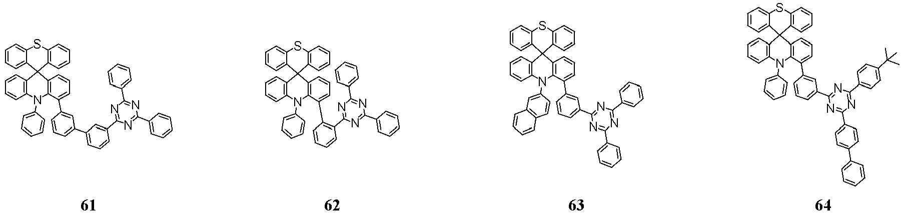

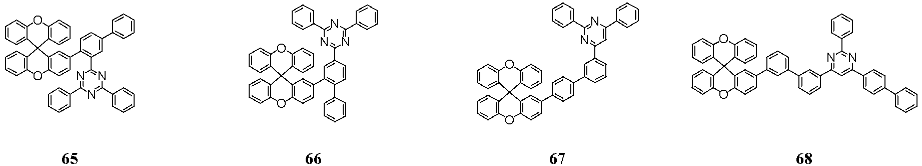

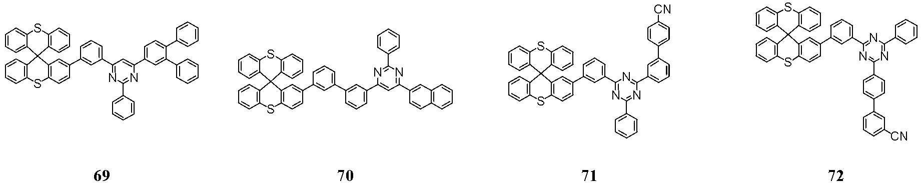

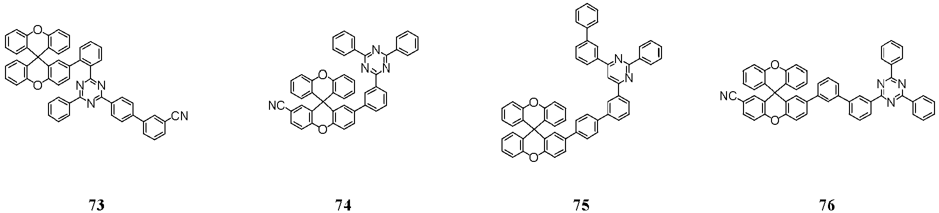

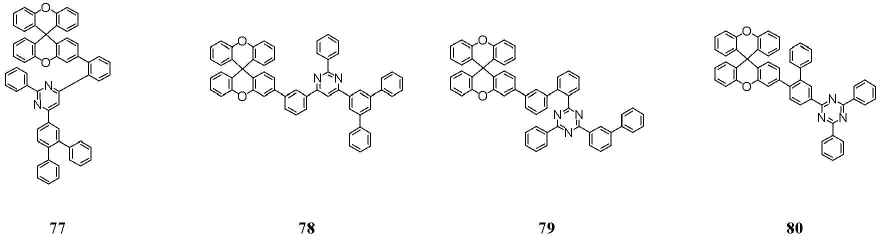

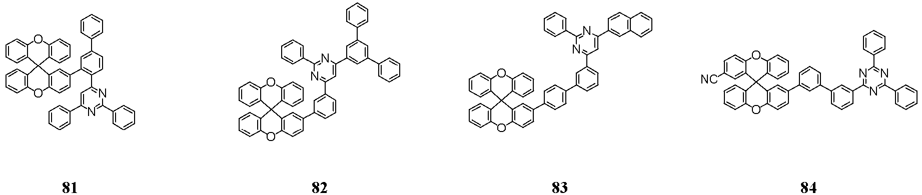

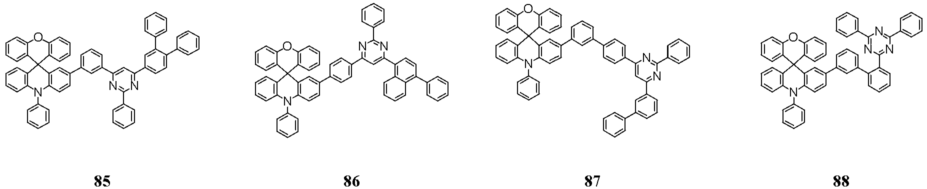

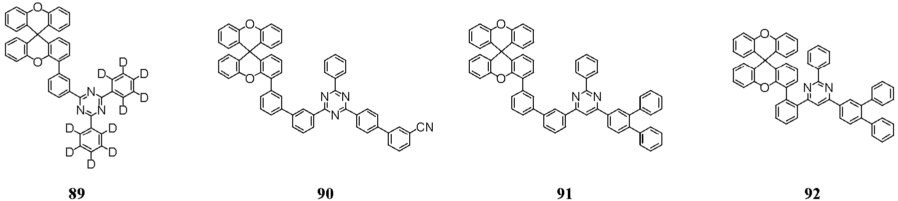

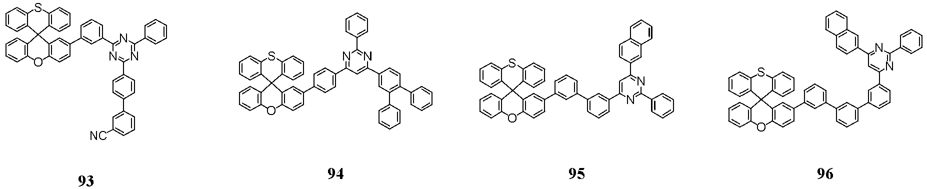

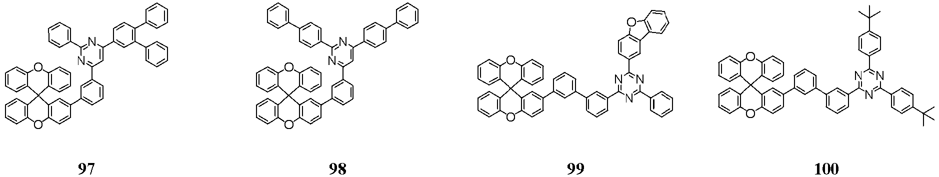

14. The compound of claim 1, wherein the compound represented by Chemical Formula 1 is used as a material of a light-emitting layer, an electron transport layer, or an auxiliary electron transport layer.

15. An organic electroluminescent device comprising:
an anode;
a cathode; and
one or more organic layers disposed between the anode and the cathode,
wherein at least one of the one or more organic layers comprises the compound according to any one of claims 1 to 14.

16. The organic electroluminescent device of claim 15, wherein the organic layer comprising the compound is selected from the group consisting of a light emitting layer, a light emitting auxiliary layer, an electron transport layer, and an auxiliary electron transport layer.
